# EUROPÄISCHE PATENTSCHRIFT

(11) **EP 1 828 137 B1**
(45) Veröffentlichungstag und Bekanntmachung des Hinweises auf die Patenterteilung: **25.04.2012**
(21) Anmeldenummer: 05820528.7
(22) Anmeldetag: 15.12.2005
(51) Int. Cl.: C07D 215/20, A61K 31/47, A61P 3/04, A61P 3/06, A61P 3/10, A61P 25/28

(54) **4-CYCLOALKYL-SUBSTITUIERTE TETRAHYDROCHINOLINDERIVATE UND DEREN VERWENDUNG ALS MEDIKAMENTE**
4-CYCLOALKYL-SUBSTITUTED TETRAHYDROCHINOLINE DERIVATIVES AND USE THEREOF AS MEDICAMENTS
DÉRIVÉS DE TÉTRAHYDROCHINOLINE SUBSTITUÉE PAR 4-CYCLOALKYLE ET LEUR UTILISATION COMME MÉDICAMENTS

(30) Priorität: 18.12.2004 DE 102004060997; 18.12.2004 DE 102004061000
(43) Veröffentlichungstag der Anmeldung: 05.09.2007
(73) Patentinhaber: Bayer Pharma Aktiengesellschaft, 13353 Berlin (DE)
(72) Erfinder: BISCHOFF, Hilmar, 42113 Wuppertal (DE); GIELEN-HAERTWIG, Heike, 40789 Monheim (DE); LI, Volkhart, 42553 Velbert (DE); SCHMECK, Carsten, 46145 Oberhausen (DE); THUTEWOHL, Michael, CH-9470 Buchs SG (CH); VAKALOPOULOS, Alexandros, 40721 Hilden (DE); WEBER, Olaf, 42489 Wülfrath (DE); WUTTKE, Martina, 42111 Wuppertal (DE)
(86) Internationale Anmeldenummer: PCT/EP2005/013490
(87) Internationale Veröffentlichungsnummer: WO 2006/063828

(56) Entgegenhaltungen:
- WO-A-99/14174
- WO-A-99/15504
- WO-A-03/028727
- US-A- 6 069 148

## Beschreibung

Die vorliegende Anmeldung betrifft ein neues Tetrahydrochinolin-Derivat, ein Verfahren zu seiner Herstellung, seine Verwendung allein oder in Kombinationen zur Behandlung und/oder Vorbeugung von Krankheiten sowie seine Verwendung zur Herstellung von Arzneimitteln, insbesondere als Inhibitor des Cholesterin-Ester-Transfer-Proteins (CETP) zur Behandlung und/oder Prävention kardiovaskulärer Erkrankungen, insbesondere von Hypolipoproteinämien, Dyslipidämien, Hypertriglyceridämien, Hyperlipidämien, Hypercholesterolämien und Arteriosklerose.

Durch Arteriosklerose bedingte koronare Herzerkrankungen gehören zu den Haupttodesursachen in der modernen Gesellschaft. In einer Vielzahl von Studien konnte gezeigt werden, dass niedrige Plasmaspiegel des HDL-Cholesterins einen wichtigen Risikofaktor für die Entwicklung von Arteriosklerose darstellen [Barter und Rye, Atherosclerosis 121, 1-12 (1996)]. HDL (high density lipoprotein) stellt neben LDL (low density lipoprotein) und VLDL (very low density lipoprotein) eine Klasse von Lipoproteinen dar, deren wichtigste Funktion der Transport von Lipiden, wie zum Beispiel Cholesterin, Cholesterinester, Triglyceriden, Fettsäuren oder Phospholipiden, im Blut ist. Hohe LDL-Cholesterinspiegel (>160 mg/dl) sowie niedrige HDL-Cholesterinspiegel (<40 mg/dl) tragen wesentlich zur Entwicklung von Arteriosklerose bei [ATP III Guidelines, Report of the NCEP Expert Panel]. Neben koronaren Herzerkrankungen werden auch periphäre Gefäßerkrankungen sowie Schlaganfall durch ungünstige HDL/LDL-Verhältnisse in ihrer Entstehung gefördert. Neue Methoden zur Erhöhung von HDL-Cholesterin im Plasma stellen demzufolge eine therapeutisch sinnvolle Bereicherung bei der Vorbeugung und Behandlung von Arteriosklerose und der damit verbundenen Krankheiten dar.

Cholesterinester-Transfer-Protein (CETP) mediiert den Austausch von Cholesterinestern und Triglyceriden zwischen den verschiedenen Lipoproteinen im Blut [Tall, J. Lipid Res. 34, 1255-74 (1993)]. Von besonderer Bedeutung ist dabei der Transfer von Cholesterinestern vom HDL auf das LDL, der zu einer Senkung der HDL-Cholesterin-Plasmaspiegel führt. Die Inhibition von CETP sollte demzufolge eine Erhöhung der Plasmaspiegel des HDL-Cholesterins und eine Absenkung der Plasmaspiegel des LDL-Cholesterins bewirken und damit zu einer therapeutisch nützlichen Beeinflussung des Lipidprofils im Plasma führen [McCarthy, Medicinal Res. Rev. 13, 139-59 (1993); Sitori, Phannac. Ther. 67, 443-47 (1995); Swenson, J. Biol. Chem. 264, 14318 (1989)].

Tetrahydrochinoline mit pharmakologischer Aktivität sind aus der EP-A-818 448, WO 99/14215, WO 99/15504 sowie WO 03/028727 bekannt. Substituierte Tetrahydronaphthaline mit pharmakologischer Aktivität sind aus der WO 99/14174 bekannt.

Aufgabe der vorliegenden Erfindung ist die Bereitstellung neuer Substanzen zur Bekämpfung von Erkrankungen, insbesondere von kardiovaskulären Erkrankungen, die ein verbessertes therapeutisches Profil aufweisen.

Gegenstand der vorliegenden Erfindung sind die Verbindungen der Strukturformel (I)

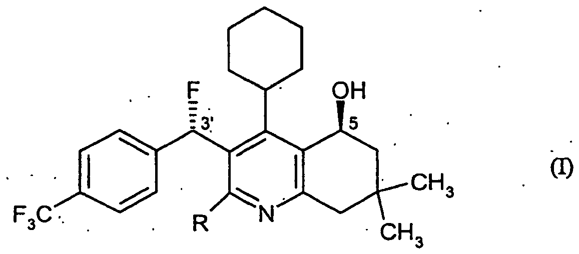

in denen R für Cyclopentyl oder iso-Propyl steht, sowie ihre Salze, Solvate und Solvate der Salze.

Gegenstand der vorliegenden Erfindung ist insbesondere die Verbindung mit dem systematischen Namen (5*S*)-4-Cyclohexyl-2-cyclopentyl-3-{(*S*)-fluor[4-(trifluormethyl)phenyl]methyl}-7,7-dimethyl-5,6,7,8-tetrahydrochinolin-5-ol und der Strukturformel (Ia) 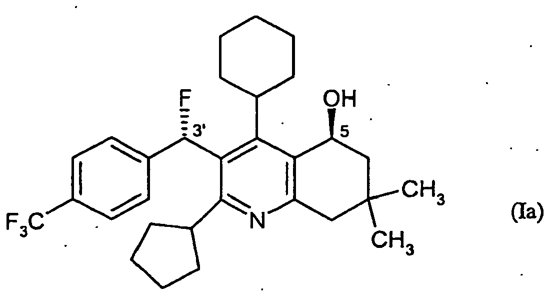 sowie ihre Salze, Solvate und Solvate der Salze.

Gegenstand der vorliegenden Erfindung ist insbesondere auch die Verbindung mit dem systematischen Namen (5*S*)-4-Cyclopentyl-3-{(*S*)-fluor[4-(trifluormethyl)phenyl]methyl}-2-isopropyl-7,7-dimethyl-5,6,7,8-tetrahydrochinolin-5-ol und der Strukturformel (Ib) 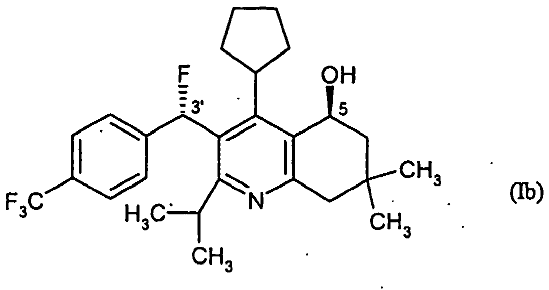 sowie ihre Salze, Solvate und Solvate der Salze.

Die Verbindungen der Formel (I) (Ia) und (Ib) werden im Folgenden im Singular als "erfindungsgemäße Verbindung" bezeichnet, die Beschreibung bezieht sich aber auf beide Verbindungen.

Die erfindungsgemäße Verbindung kann auch in anderen stereoisomeren Formen (Enantiomere, Diastereomere) vorliegen. Die vorliegende Erfindung umfasst alle Enantiomeren, Diastereomeren und ihre jeweiligen Mischungen. Aus solchen Mischungen von Enantiomeren und/oder Diastereomeren lassen sich die stereoisomer einheitlichen Bestandteile in bekannter Weise isolieren. Bevorzugt ist die in Formel (I) wiedergegebene S-Konfiguration an C-5 und an C-3'.

Als Salze sind im Rahmen der vorliegenden Erfindung physiologisch unbedenkliche Salze der erfindungsgemäßen Verbindung bevorzugt. Umfasst sind auch Salze, die für pharmazeutische Anwendungen selbst nicht geeignet sind, jedoch beispielsweise für die Isolierung oder Reinigung der erfindungsgemäßen Verbindung verwendet werden können.

Physiologisch unbedenkliche Salze der erfindungsgemäßen Verbindung umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindung umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindung bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

(C₁-C₄)-Alkyl steht im Rahmen der Erfindung für einen geradkettigen oder verzweigten Alkylrest mit 1 bis 4 Kohlenstoffatomen. Beispielhaft und vorzugsweise seien genannt: Methyl, Ethyl, n-Propyl, Isopropyl, n-Butyl, iso-Butyl, sec.-Butyl und tert.-Butyl.

Weiterer Gegenstand der Erfindung ist ein Verfahren zur Herstellung der erfindungsgemäßen Verbindung der Formel (Ia), dadurch gekennzeichnet, dass man die Verbindung der Formel (IIa) 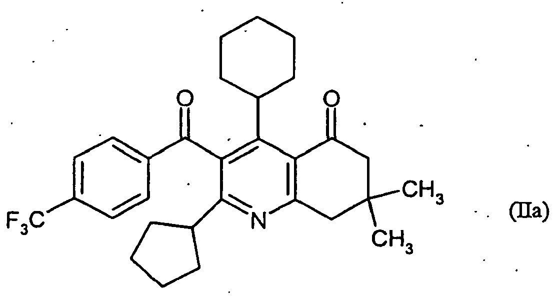 zunächst durch eine asymmetrische Reduktion in die Verbindung der Formel (IIIa) 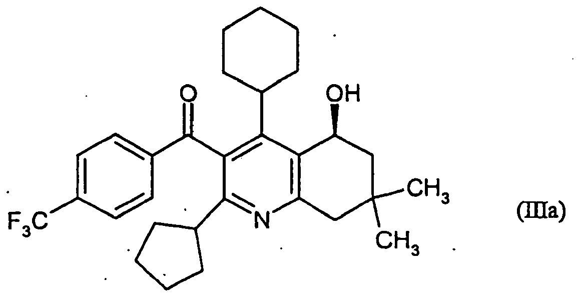 überführt, diese dann entweder

### [A] durch Einführung einer Hydroxy-Schutzgruppe zu einer Verbindung der Formel (IVa)

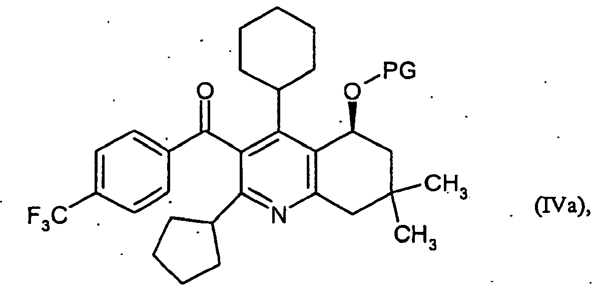

in welcher
PG für eine Hydroxy-Schutzgruppe, vorzugsweise für einen Rest der Formel -SiR¹R²R³ steht, worin
   R¹, R² und R³ gleich oder verschieden sind und (C₁-C₄)-Alkyl bedeuten,
umsetzt und anschließend über eine diastereoselektive Reduktion in eine Verbindung der Formel (Va) 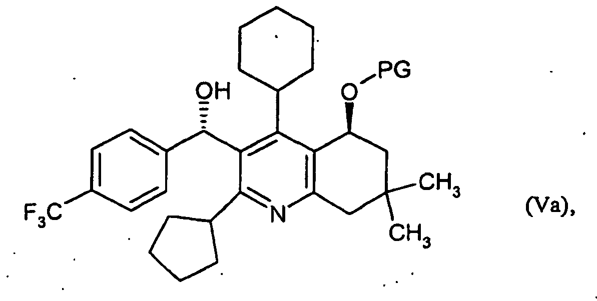 in welcher PG die oben angegebene Bedeutung hat, überführt
oder in umgekehrter Abfolge der Reaktionssequenz

### [B] zunächst diastereoselektiv zu der Verbindung der Formel (VIa)

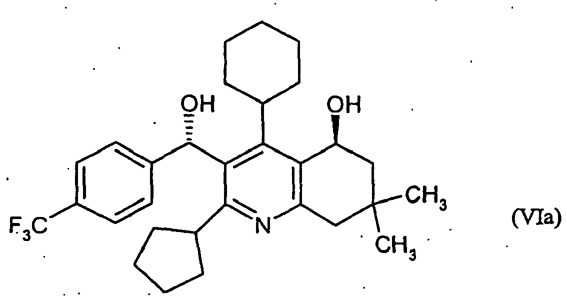

reduziert und diese dann durch regioselektive Einführung der Hydroxy-Schutzgruppe PG in eine Verbindung der Formel (V) überführt,
anschließend die Verbindung der Formel (Va) mit Hilfe eines Fluorierungsmittels zu einer Verbindung der Formel (VIIa) 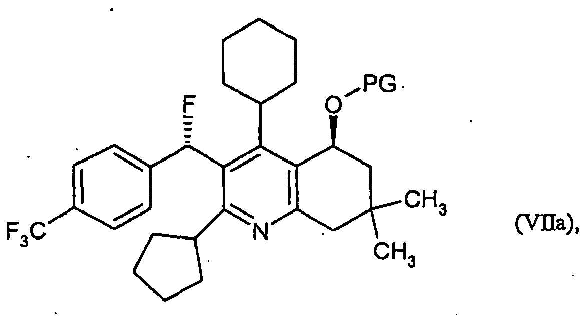 in welcher PG die oben angegebene Bedeutung hat,
umsetzt und dann die Hydroxy-Schutzgruppe PG nach üblichen Methoden unter Erhalt der Verbindung der Formel (Ia) wieder abspaltet
und die Verbindung der Formel (Ia) gegebenenfalls mit den entsprechenden (i) Lösungsmitteln und/oder (ii) Basen oder Säuren zu ihren Solvaten, Salzen und/oder Solvaten der Salze umsetzt.

Die Verbindung der Formel (IIa) kann hergestellt werden, indem man die Verbindungen der Formeln (VIII), (IX) und (Xa) 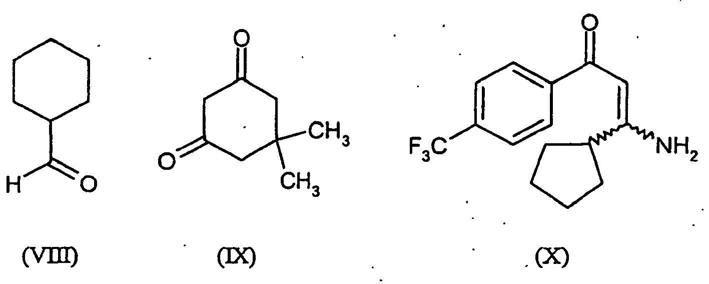 miteinander in einer 3-Komponenten-Reaktion in Gegenwart einer Protonen- oder Lewis-Säure zu der Verbindung der Formel (XIa) 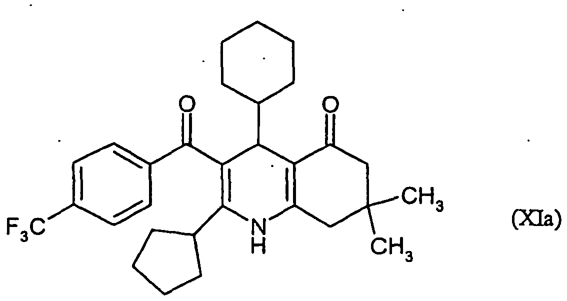 umsetzt und diese dann zu der Verbindung der Formel (IIa) oxidiert.

Weiterer Gegenstand der Erfindung ist ein Verfahren zur Herstellung der erfindungsgemäßen Verbindung der Formel (Ib), dadurch gekennzeichnet, dass man die Verbindung der Formel (IIb) 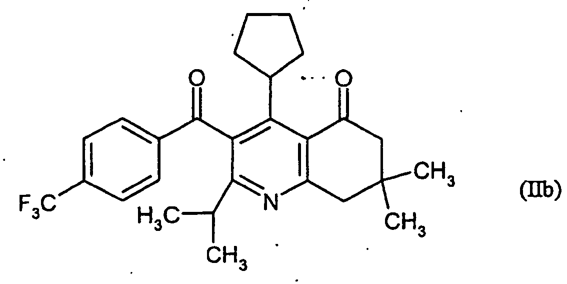 zunächst durch eine asymmetrische Reduktion in die Verbindung der Formel (IIIb) 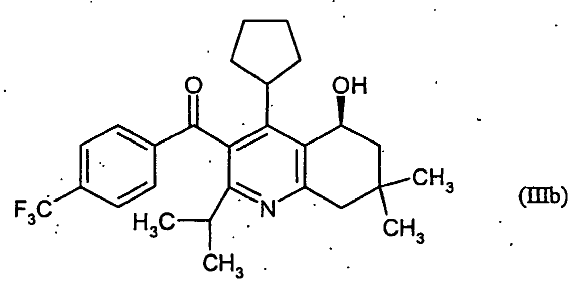 überführt, diese dann entweder

### [A] durch Einführung einer Hydroxy-Schutzgruppe zu einer Verbindung der Formel (IVb)

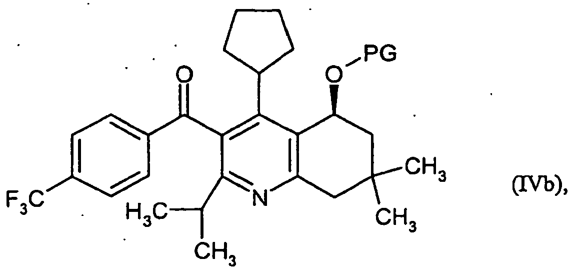

in welcher
PG für eine Hydroxy-Schutzgruppe, vorzugsweise für einen Rest der Formel -SiR¹R²R³ steht, worin
   R¹, R² und R³ gleich oder verschieden sind und (C₁-C₄)-Alkyl bedeuten,
umsetzt und anschließend über eine diastereoselektive Reduktion in eine Verbindung der Formel (Vb) 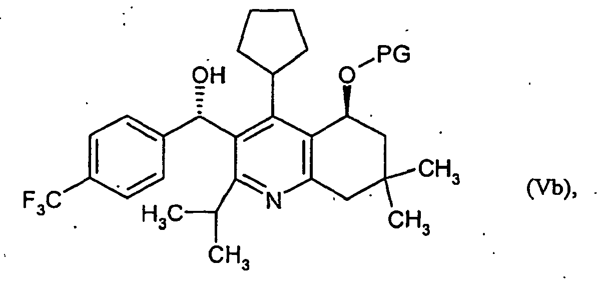 in welcher PG die oben angegebene Bedeutung hat, überführt
oder in umgekehrter Abfolge der Reaktionssequenz

### [B] zunächst diastereoselektiv zu der Verbindung der Formel (VIb)

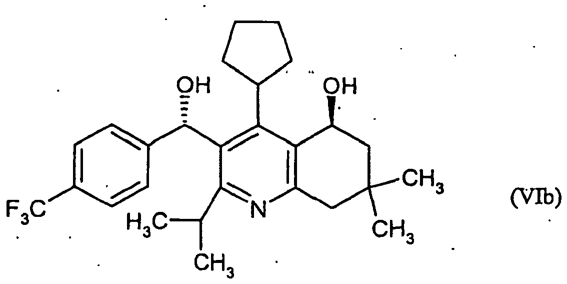

reduziert und diese dann durch regioselektive Einführung der Hydroxy-Schutzgruppe PG in eine Verbindung der Formel (Vb) überführt,
anschließend die Verbindung der Formel (Vb) mit Hilfe eines Fluorierungsmittels zu einer Verbindung der Formel (VIIb) 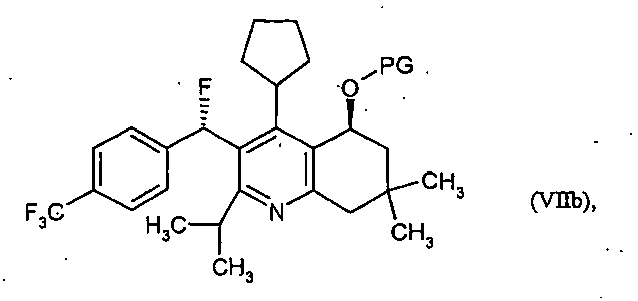 in welcher PG die oben angegebene Bedeutung hat,
umsetzt und dann die Hydroxy-Schutzgruppe PG nach üblichen Methoden unter Erhalt der Verbindung der Formel (Ib) wieder abspaltet
und die Verbindung der Formel (Ib) gegebenenfalls mit den entsprechenden (i) Lösungsmitteln und/oder (ii) Basen oder Säuren zu ihren Solvaten, Salzen und/oder Solvaten der Salze umsetzt.

Die Verbindung der Formel (IIb) kann hergestellt werden, indem man die Verbindungen der Formeln (VIII), (IX) und (Xb) 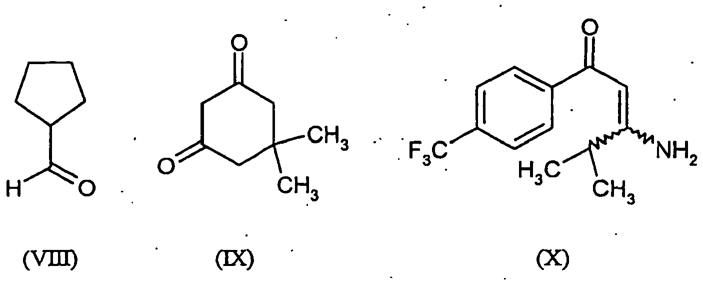 miteinander in einer 3-Komponenten-Reaktion in Gegenwart einer Protonen- oder Lewis-Säure zu der Verbindung der Formel (XIb) 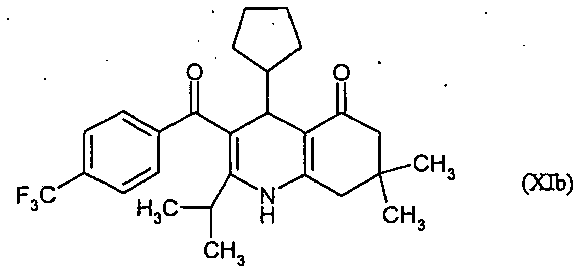 umsetzt und diese dann zu der Verbindung der Formel (IIb) oxidiert.

Die Verbindungen der Formeln (VIII), (IX) und (Xa) und (Xb) sind kommerziell erhältlich, literaturbekannt oder können in Analogie zu literaturbekannten Verfahren hergestellt werden (vgl. auch WO 99/14215 und WO 03/028727).

Als inerte Lösungsmittel für die einzelnen Verfahrensschritte sind beispielsweise Ether wie Diethylether, Diisopropylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, 1,2-Dichlorethan, Trichlorethylen oder Chlorbenzol geeignet. Ebenso ist es möglich, Gemische der genannten Lösungsmittel zu verwenden.

Die Reduktionen in den Verfahrensschritten (II) → (III), (IV) → (V) und (III) → (VI) werden im Allgemeinen mit Reduktionsmitteln, die für die Reduktion von Kefonen zu Hydroxyverbindungen geeignet sind, durchgeführt. Hierzu gehören insbesondere komplexe Aluminium- oder Borhydride wie beispielsweise Lithium-, Natrium-, Kalium-, Zinkborhydrid, Lithiumaluminiumhydrid, Diisobutylaluminiumhydrid (DIBAH), Natrium-bis-(2-methoxyethoxy)-aluminiumdihydrid, Lithiumtrialkylborhydride oder Lithiumtrialkoxyaluminiumhydride, oder Boran-Komplexe wie beispielsweise Boran-Tetrahydrofuran-, Boran-Dimethylsulfid- oder Boran-*N,N*-Diethylanilin-Komplex.

Die asymmetrische Reduktion im Verfahrensschritt (II) → (III) erfolgt in Gegenwart katalytischer Mengen (0.01 bis 0.3 Mol-Äquivalente) von enantiomerenreinem (1*R*,2*S*)-1-Aminoindan-2-ol als chiralem Induktor. Als Reduktionsmittel wird hierbei bevorzugt Boran-NN-Diethylanilin-Komplex verwendet. Die Reaktion wird im Allgemeinen in einem der oben aufgeführten Ether oder in Toluol, vorzugsweise in Tetrahydrofuran, in einem Temperaturbereich von -80°C bis +50°C, bevorzugt von 0°C bis +30°C, durchgeführt.

Bei den Reduktionen (IV) → (V) und (III) → (VI) wird bevorzugt Lithiumaluminiumhydrid bzw. DIBAH als Reduktionsmittel verwendet. Die Reaktionen werden im Allgemeinen in einem der oben aufgeführten Ether oder in Toluol, vorzugsweise in Tetrahydrofuran oder Toluol, in einem Temperaturbereich von -80°C bis +50°C, bevorzugt von 0°C bis +30°C im Falle von Lithiumaluminiumhydrid und von -80°C bis +30°C im Falle von DIBAH, durchgeführt.

Als Hydroxy-Schutzgruppe bei den Verfahrensschritten (III) → (IV) bzw. (VI) → (V) wird eine Silylgruppe, wie beispielsweise Trimethylsilyl, Triethylsilyl, Triisopropylsilyl oder *tert.*-Butyldimethylsilyl, bevorzugt. Besonders bevorzugt ist *tert.*-Butyldimethylsilyl. Die Einführung der Silylgruppe erfolgt im Allgemeinen in einem der oben aufgeführten Kohlenwasserstoffe, Halogenkohlenwasserstoffe, Ether oder in Dimethylformamid als Lösungsmittel in Gegenwart einer Base wie beispielsweise Triethylamin, *N,N*-Diisopropylethylamin, Pyridin, 2,6-Lutidin oder 4-*N,N*-Dimethylaminopyridin (DMAP).

Im Verfahrensschritt (III) → (IV) wird bevorzugt *tert.*-Butyldimethylsilyltrifluormethansulfonat als Silylierungsreagenz in Kombination mit 2,6-Lutidin als Base verwendet. Die Reaktion wird bevorzugt in Dichlormethan oder Toluol in einem Temperaturbereich von -40°C bis +40°C, bevorzugt von -20°C bis +30°C, durchgeführt.

Im Verfahrensschritt (VI) → (V) wird bevorzugt *tert.*-Butyldimethylsilylchlorid als Silylierungsreagenz in Kombination mit Triethylamin und DMAP als Basen verwendet. Die Reaktion wird bevorzugt in Dimethylformamid in einem Temperaturbereich von 0°C bis +100°C, bevorzugt von +20°C bis +80°C, durchgeführt.

Die Fluorierung im Verfahrensschritt (VI) → (VII) wird im Allgemeinen in einem der oben aufgeführten Kohlenwasserstoffe oder Halogenkohlenwasserstoffe oder in Acetonitril, vorzugsweise in Toluol oder Dichlormethan, mit Diethylaminoschwefeltrifluorid (DAST) oder Morpholinoschwefeltrifluorid als Fluorierungsreagenz durchgeführt. Die Reaktion erfolgt im Allgemeinen in einem Temperaturbereich von -80°C bis +40°C, bevorzugt von -60°C bis +20°C.

Die Abspaltung einer Silyl-Schutzgruppe im Verfahrensschritt (VII) → (I) wird im Allgemeinen mit Hilfe von Säuren, wie beispielsweise Salzsäure oder Trifluoressigsäure, oder mit Hilfe von Fluoriden, wie beispielsweise Fluorwasserstoff oder Tetrabutylammoniumfluorid (TBAF), durchgeführt. Als inerte Lösungsmittel sind die oben aufgeführten Ether, Alkohole wie Methanol oder Ethanol, oder Gemische der genannten Lösungsmittel geeignet. Bevorzugt ist eine Abspaltung mit TBAF in Tetrahydrofuran als Lösungsmittel. Die Reaktion erfolgt im Allgemeinen in einem Temperaturbereich von -20°C bis +60°C, bevorzugt von 0°C bis +30°C.

Die Kondensationsreaktion (VIII) + (IX) + (X) → (XI) wird im Allgemeinen in einem der oben aufgeführten Ether, in Alkoholen wie Methanol, Ethanol, n-Propanol oder Isopropanol, in Acetonitril oder in Gemischen der genannten Lösungsmittel durchgeführt. Bevorzugt wird Diisopropylether verwendet.

Als Protonen-Säuren eignen sich für diesen Verfahrensschritt im Allgemeinen organische Säuren, wie beispielsweise Essigsäure, Trifluoressigsäure, Oxalsäure oder para-Toluolsulfonsäure, oder anorganische Säuren, wie beispielsweise Salzsäure, Schwefelsäure oder Phosphorsäure. Ebenfalls geeignet sind Lewis-Säuren wie beispielsweise Aluminiumchlorid oder Zinkchlorid. Bevorzugt ist Trifluoressigsäure.

Die Reaktion erfolgt im Allgemeinen in einem Temperaturbereich von 0°C bis +120°C, bevorzugt von +20°C bis +80°C.

Die Oxidation (Dehydrierung) im Verfahrensschritt (XI) → (II) wird im Allgemeinen in einem der oben aufgeführten Halogenkohlenwasserstoffe oder gegebenenfalls in Alkoholen wie Methanol oder Ethanol, in Acetonitril oder in Wasser durchgeführt. Als Oxidationsmittel eignen sich beispielsweise Salpetersäure, Cer(IV)-ammoniumnitrat, 2,3-Dichlor-5,6-dicyano-1,4-benzochinon (DDQ), Pyridiniumchlorochromat (PCC), Osmiumtetroxid, Mangandioxid oder eine katalytische Dehydrierung mittels Platindioxid oder Palladium auf Aktivkohle. Bevorzugt ist eine Oxidation mit DDQ in Dichlormethan als Lösungsmittel. Die oxidation erfolgt im Allgemeinen in einem Temperaturbereich von -50°C bis +100°C, bevorzugt von 0°C bis +40°C.

Die einzelnen Verfahrensschritte können bei normalem, erhöhtem oder bei erniedrigtem Druck durchgeführt werden (z.B. von 0.5 bis 5 bar). Im Allgemeinen arbeitet man bei Normaldruck.

Die Herstellung der erfindungsgemäßen Verbindung kann durch das folgende Syntheseschema veranschaulicht werden: 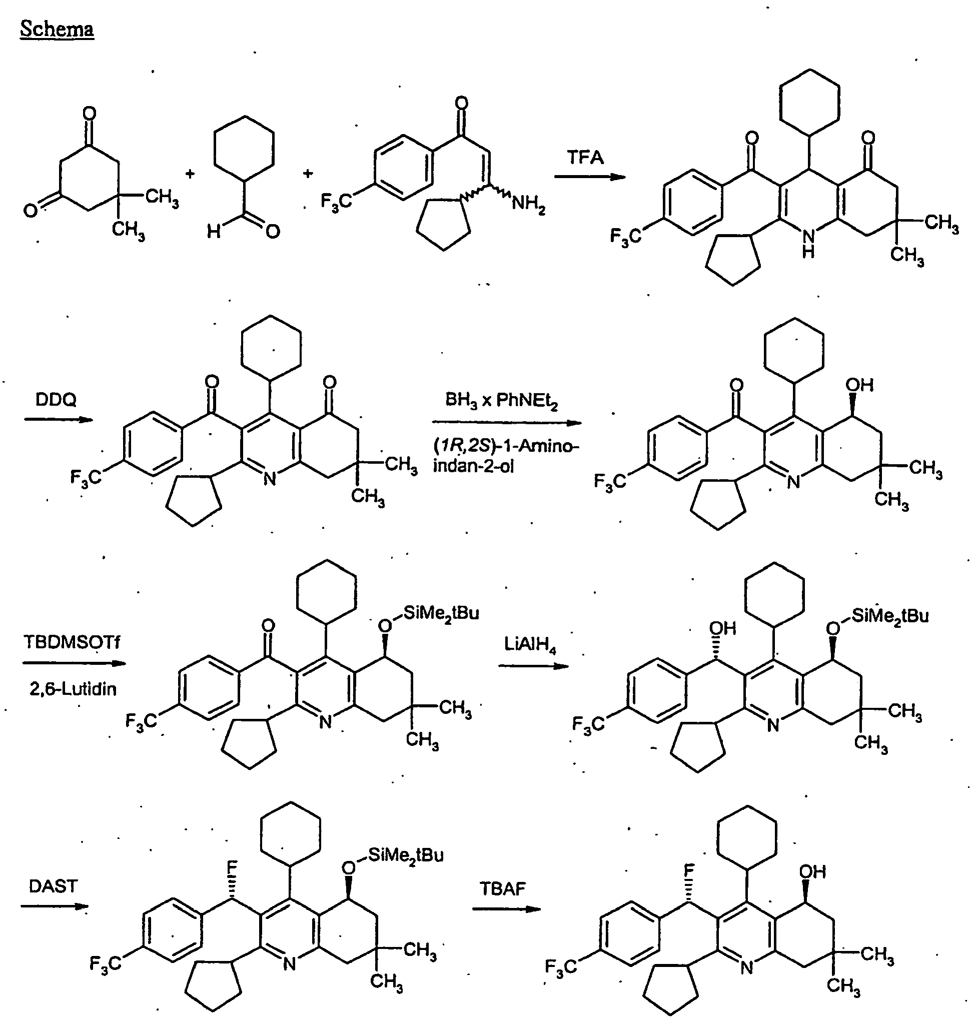 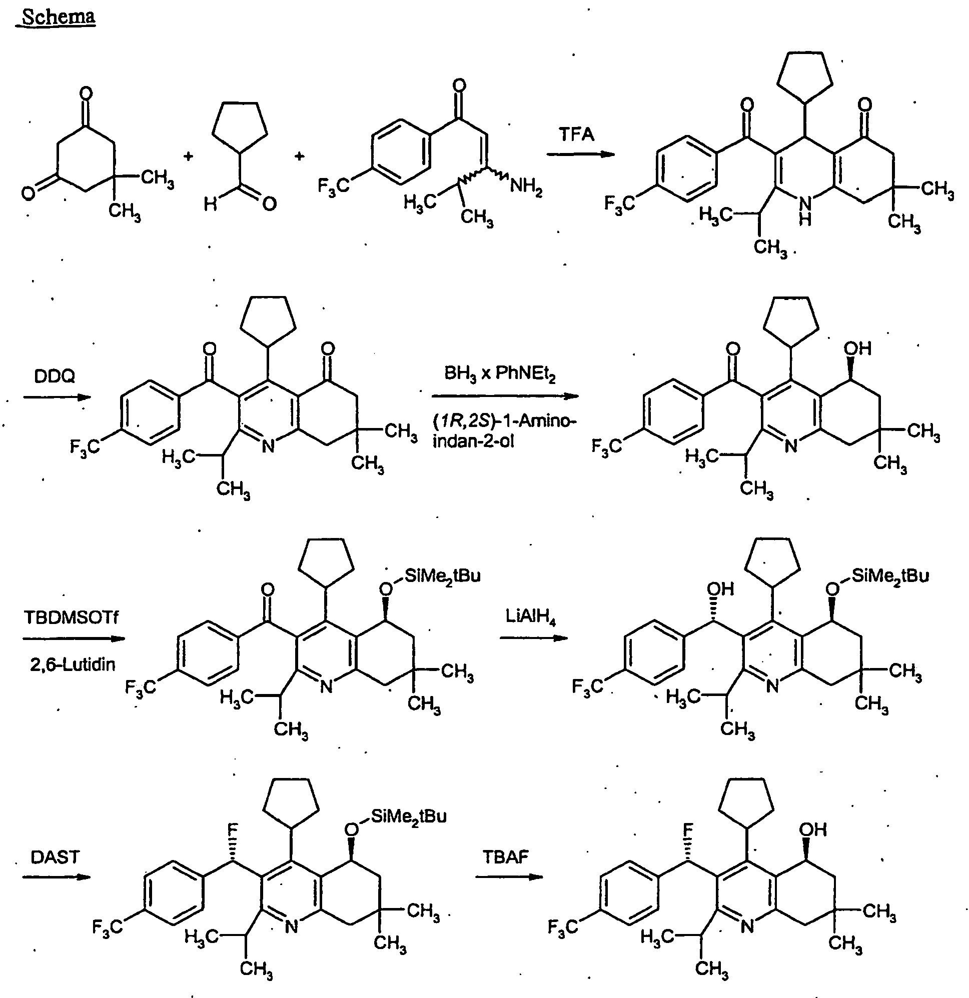 [Abkürzungen: tBu = *tert.*-Butyl; DAST = Dimethylaminoschwefeltrifluorid; DDQ = 2,3-Dichlor-5,6-dicyano-1,4-benzochinon; Et = Ethyl; Me = Methyl; Ph = Phenyl; TBAF = Tetrabutylammoniumfluorid; TBDMSOTf = *tert.*-Butyldimethylsilyl-trifluormethansulfonat; TFA = Trifluoressigsäure].

Die erfindungsgemäße Verbindung zeigt ein nicht vorhersehbares, wertvolles pharmakologisches Wirkspektrum. Sie eignet sich daher zur Verwendung als Arzneimittelwirkstoff zur Behandlung und/oder Prophylaxe von Krankheiten bei Menschen und Tieren.

Die erfindungsgemäße Verbindung eröffnet eine weitere Behandlungsalternative und stellt eine Bereicherung der Pharmazie dar. Im Vergleich zu den bekannten und bisher eingesetzten Präparaten zeigt die erfindungsgemäße Verbindung ein verbessertes Wirkungsspektrum.

Sie zeichnet sich vorzugsweise durch große Spezifität, gute Verträglichkeit und geringere Nebenwirkungen sowie eine geringere Toxizität insbesondere im Herz-Kreislauf-Bereich und im Bereich der Leber aus.

Ein Vorteil der erfindungsgemäßen Verbindung ist ihre hohe Aktivität in humanem Plasma. Ein weiterer Vorteil der erfindungsgemäßen Verbindung ist ein verringertes Interaktionspotential mit metabolisierenden Enzymen, insbesondere mit den Cytochrom P450-Enzymen und in besonderem Masse mit dem Cytochrom P450 3A4-Enzym. Die erfindungsgemäße Verbindung zeigt darüber hinaus ein verringertes Ablagerungsverhalten im Fettgewebe.

Die erfindungsgemäße Verbindung der Formel (I) besitzt wertvolle pharmakologische Eigenschaften und kann zur Vorbeugung und Behandlung vor Erkrankungen verwendet werden. Insbesondere ist die erfindungsgemäße Verbindung ein hochwirksamer Inhibitor des Cholesterin-Ester-Transfer-Proteins (CETP) und stimuliert den reversen Cholesterin-Transport. Sie bewirkt eine Erhöhung des HDL-Cholesterinspiegels im Blut. Die erfmdungsgemäße Verbindung ist insbesondere zur Behandlung und zur primären oder sekundären Prävention koronarer Herzerkrankungen, z.B. von Myokardinfarkt, einsetzbar. Die erfindungsgemäße Verbindung kann zudem zur Behandlung und Prävention von Arteriosklerose, Restenose, Schlaganfällen sowie der Alzheimer'schen Krankheit verwendet werden. Darüber hinaus kann die erfindungsgemäße Verbindung auch zur Behandlung und Prävention von Hypolipoproteinämien, Dyslipidämien, Hypertriglyceridämien, Hyperlipidämien, Hypercholesterolämien, Fettsucht (Adipositas), Fettleibigkeit (Obesitas), Pankreatitis, Insulin-abhängigem und nicht-Insulin-abhängigem Diabetes, diabetischen Spätfolgen, wie beispielsweise Retinopathie, Nephropathie und Neuropathie, von kombinierten Hyperlipidämien sowie des Metabolischen Syndroms eingesetzt werden.

Die pharmakologische Wirkung der erfindungsgemäßen Verbindung kann mittels der im Folgenden aufgeführten CETP-Inhibitions-Tests ermittelt werden.

Weiterer Gegenstand der vorliegenden Erfindung ist die Verwendung der erfindungsgemäßen Verbindung zur Herstellung von Medikamenten zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindung zur Herstellung eines Arzneimittels zur Behandlung und/oder Prävention von Erkrankungen, insbesondere der zuvor genannten Erkrankungen.

Weiterer Gegenstand der vorliegenden Erfindung sind Arzneimittel, die die erfindungsgemäße Verbindung sowie einen oder mehrere weitere Wirkstoffe enthalten, zur Behandlung und/oder Prävention von Erkrankungen. Als geeignete Kombinationswirkstoffe seien beispielhaft und vorzugsweise genannt:
- Antidiabetika,
- antithrombotisch wirkende Substanzen,
- blutdrucksenkende Substanzen,
- den Fettstoffwechsel verändernde Substanzen,
- antientzündlich wirkende Substanzen,
- den arteriosklerotischen Plaque stabilisierende Substanzen.

Die erfindungsgemäße Verbindung der Formel (I) kann vorzugsweise mit einem oder mehreren
- Antidiabetika, die in der Roten Liste 2002/II, Kapitel 12 genannt sind,
- antithrombotisch wirkenden Mitteln, beispielhaft und vorzugsweise aus der Gruppe der Thrombozytenaggregationshemmer oder der Antikoagulantien,
- den Blutdruck senkenden Wirkstoffen, beispielhaft und vorzugsweise aus der Gruppe der Calcium-Antagonisten, Angiotensin AII-Antagonisten, ACE-Hemmer, beta-Blocker, Phosphodiesterase-Inhibitoren, Stimulatoren der löslichen Guanylatcyclase, cGMP-Enhancer sowie der Diuretika, und/oder
- den Fettstoffwechsel verändernden Wirkstoffen, beispielhaft und vorzugsweise aus der Gruppe der Thyroidrezeptor-Agonisten, der Cholesterinsynthese-Inhibitoren wie HMG-CoA-Reduktase-Inhibitoren, Squalensynthase-Inhibitoren, Squalenepoxidase-Inhibitoren oder Oxidosqualencyclase-Inhibitoren, der ACAT-Inhibitoren, MTP-Inhibitoren, PPAR-Agonisten, Fibrate, Lipase-Inhibitoren, Cholesterinabsorptionshemmer, Gallensäure-Reabsorptionshemmer, polymeren Gallensäureadsorber und der Lipoprotein(a)-Antagonisten
kombiniert werden.

Unter Antidiabetika werden beispielhaft und vorzugsweise Insulin und Insulinderivate sowie oral wirksame hypoglykämische Wirkstoffe verstanden.

Insulin und Insulinderivate umfasst hierbei sowohl Insuline tierischen, menschlichen oder biotechnologischen Ursprungs als auch Gemische hieraus.

Die oral wirksamen hypoglykämischen Wirkstoffe umfassen beispielhaft und vorzugsweise Sulphonylharnstoffe, Biguadine, Meglitinid-Derivate, Oxadiazolidinone, Thiazolidindione, Glukosidase-Inhibitoren, Glukagon-Antagonisten, GLP-1-Agonisten, Insulin-Sensitizer, Inhibitoren von Leberenzymen, die an der Stimulation der Glukoneogenese und/oder Glykogenolyse beteiligt sind, Modulatoren der Glukoseaufnahme sowie Kaliumkanalöffner, wie z.B. diejenigen, die in WO 97/26265 und WO 99/03861 offenbart sind.

Bei einer bevorzugten Ausführungsform der Erfindung wird die Verbindung der Formel (I) in Kombination mit Insulin verabreicht.

Bei einer bevorzugten Ausführungsform der Erfindung wird die Verbindung der Formel (I) in Kombination mit einem Sulphonylharnstoff, wie beispielhaft und vorzugsweise Tolbutamid, Glibenclamid, Glimepirid, Glipizid oder Gliclazid, verabreicht.

Bei einer bevorzugten Ausführungsform der Erfindung wird die Verbindung der Formel (I) in Kombination mit einem Biguanid, wie beispielhaft und vorzugsweise Metformin, verabreicht.

Bei einer bevorzugten Ausführungsform der Erfindung wird die Verbindung der Formel (I) in Kombination mit einem Meglitinid-Derivat, wie beispielhaft und vorzugsweise Repaglinid oder Nateglinid, verabreicht.

Bei einer bevorzugten Ausführungsform der Erfindung wird die Verbindung, der Formel (I) in Kombination mit einem PPARgamma-Agonisten beispielsweise aus der Klasse der Thiazolidindione, wie beispielhaft und vorzugsweise Pioglitazon oder Rosiglitazon, verabreicht.

Bei einer bevorzugten Ausführungsform der Erfindung wird die Verbindung der Formel (I) in Kombination mit einem gemischten PPARalpha/gamma-Agonisten, wie beispielhaft und vorzugsweise GI-262570 (Farglitazar), GW 2331, GW 409544, AVE 8042, AVE 8134, AVE 0847, MK-0767 (KRP-297) oder AZ-242, verabreicht.

Unter antithrombotisch wirkenden Mitteln werden vorzugsweise Verbindungen aus der Gruppe der Thrombozytenaggregationshemmer, wie beispielhaft und vorzugsweise Aspirin, Clopidogrel, Ticlopidin oder Dipyridamol, oder der Antikoagulantien verstanden.

Bei einer bevorzugten Ausführungsform der Erfindung wird die Verbindung der Formel (I) in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung wird die Verbindung der Formel (I) in Kombination mit einem GPIIb/IIIa-Antagonisten, wie beispielhaft und vorzugsweise Tirofiban oder Abciximab, verabreicht.

Bei einer bevorzugten Ausführungsform der Erfindung wird die Verbindung der Formel (I) in Kombination mit einem Faktor Xa-Inhibitor, wie beispielhaft und vorzugsweise DX 9065a, DPC 906, JTV 803 oder BAY 59-7939, verabreicht.

Bei einer bevorzugten Ausführungsform der Erfindung wird die Verbindung der Formel (I) in Kombination mit Heparin oder einem low molecular weight (LMW)-Heparin-Derivat verabreicht.

Bei einer bevorzugten Ausführungsform der Erfindung wird die Verbindung der Formel (I) in Kombination mit einem Vitamin K-Antagonisten, wie beispielhaft und vorzugsweise Coumarin, verabreicht.

Unter blutdrucksenkenden Mitteln werden beispielhaft und vorzugsweise Verbindungen aus der Gruppe der Calcium-Antagonisten, wie beispielhaft und vorzugsweise die Verbindungen Nifedipin, Amlodipin, Nitrendipin, Nisoldipin, Verapamil oder Diltiazem, der Angiotensin AII-Antagonisten, ACE-Hemmer, beta-Blocker sowie der Diuretika verstanden.

Bei einer bevorzugten Ausführungsform der Erfindung wird die Verbindung der Formel (I) in Kombination mit einem Antagonisten der alpha 1-Rezeptoren verabreicht.

Bei einer bevorzugten Ausführungsform der Erfindung wird die Verbindung der Formel (I) in Kombination mit Reserpin, Minoxidil, Diazoxid, Dihydralazin, Hydralazin sowie Stickoxid freisetzenden Stoffen, wie beispielhaft und vorzugsweise Glycerinnitrat oder Nitroprussidnatrium, verabreicht.

Bei einer bevorzugten Ausführungsform, der Erfindung wird die Verbindung der Formel (I) in Kombination mit einem Angiotensin AII-Antagonisten, wie beispielhaft und vorzugsweise Losartan, Valsartan, Candesartan, Telmisartan, Embusartan, Irbesartan, Olmesartan, Tasosartan oder Saprisartan, verabreicht.

Bei einer bevorzugten Ausführungsform der Erfindung wird die Verbindung der Formel (I) in Kombination mit einem ACE-Hemmer, wie beispielhaft und vorzugsweise Enalapril, Captopril, Ramipril, Delapril, Fosinopril, Quinopril, Perindopril oder Trandolapril, verabreicht.

Bei einer bevorzugten Ausführungsform der Erfindung wird die Verbindung der Formel (I) in Kombination mit einem beta-Blocker, wie beispielhaft und vorzugsweise Propranolol oder Atenolol, verabreicht.

Bei einer bevorzugten Ausführungsform der Erfindung wird die Verbindung der Formel (I) in Kombination mit einem Diuretikum, wie beispielhaft und vorzugsweise Furosemid, verabreicht.

Unter den Fettstoffwechsel verändernden Mitteln werden beispielhaft und vorzugsweise Verbindungen aus der Gruppe der Thyroidrezeptor-Agonisten, der Cholesterinsynthese-Inhibitoren wie HMG-CoA-Reduktase-Inhibitoren oder Squalensynthese-Inhibitoren, der ACAT-Inhibitoren, MTP-Inhibitoren, PPAR-Agonisten, Fibrate, Cholesterinabsorptionshemmer, Gallensäure-Reabsorptionshemmer, Lipase-Inhibitoren, polymeren Gallensäureadsorber sowie der Lipoprotein(a)-Antagonisten verstanden.

Bei einer bevorzugten Ausführungsform der Erfindung wird die Verbindung der Formel (I) in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung wird die Verbindung der Formel (I) in Kombination mit einem Squalensynthese-Inhibitor, wie beispielhaft und vorzugsweise BMS-188494 oder TAK 475, verabreicht.

Bei einer bevorzugten Ausführungsform der Erfindung wird die Verbindung der Formel (I) in Kombination mit einem ACAT-Inhibitor, wie beispielhaft und vorzugsweise Avasimibe, Eflucimibe oder CS-505, verabreicht.

Bei einer bevorzugten Ausführungsform der Erfindung wird die Verbindung der Formel (I) in Kombination mit einem Cholesterinabsorptionshemmer, wie beispielhaft und vorzugsweise Ezetimibe, Tiqueside oder Pamaqueside, verabreicht.

Bei einer bevorzugten Ausführungsform der Erfindung wird die Verbindung der Formel (I) in Kombination mit einem Gallensäure-Reabsorptionshemmer, wie beispielhaft und vorzugsweise Barixibat, AZD 7508, SC 435, SC 635, S-8921, 264W94 oder HM 1453, verabreicht.

Bei einer bevorzugten Ausführungsform der Erfindung wird die Verbindung der Formel (I) in Kombination mit einem MTP-Inhibitor, wie beispielhaft und vorzugsweise Implitapide, BMS-201038 oder R-103757, verabreicht.

Bei einer bevorzugten Ausführungsform der Erfindung wird die Verbindung der Formel (I) in Kombination mit einem PPARalpha-Agonisten, wie z.B. die Fibrate Fenofibrat, Clofibrat, Bezafibrat, Ciprofibrat oder Gernfibrozil oder wie beispielhaft und vorzugsweise GW 9578, GW 7647, LY-518674 oder NS-220, verabreicht.

Bei einer bevorzugten Ausführungsform der Erfindung wird die Verbindung der Formel (I) in Kombination mit einem PPARdelta-Agonisten, wie beispielhaft und vorzugsweise GW 501516, verabreicht.

Bei einer bevorzugten Ausführungsform der Erfindung wird die Verbindung der Formel (I) in Kombination mit einem gemischten PPARalpha/gamma-Agonisten, wie beispielhaft und vorzugsweise GI-262570 (Farglitazar), GW 2331, GW 409544, AVE 8042, AVE 8134, AVE 0847, MK-0767 (KRP-297) oder AZ-242, verabreicht.

Bei einer bevorzugten Ausführungsform der Erfindung wird die Verbindung der Formel (I) in Kombination mit einem gemischten PPARalpha/gamma/delta-Agonisten, wie beispielhaft und vorzugsweise MCC-555, verabreicht.

Bei einer bevorzugten Ausführungsform der Erfindung wird die Verbindung der Formel (I) in Kombination mit einem Lipase-Inhibitor aus der Gruppe der endothelialen Lipase-Inhibitoren, der pankreatischen Lipase-Inhibitoren, der gastrischen Lipase-Inhibitoren, der Hormon-sensitiven Lipase-Inhibitoren oder der hepatischen Lipase-Inhibitoren verabreicht.

Bei einer besonders bevorzugten Ausführungsform der Erfindung wird die Verbindung der Formel (I) in Kombination mit einem Inhibitor der pankreatrischen Lipase, vorzugsweise aus der Klasse der Lipstatine wie beispielhaft Orlistat, verabreicht.

Bei einer bevorzugten Ausführungsform der Erfindung wird die Verbindung der Formel (I) in Kombination mit einem polymeren Gallensäureadsorber, wie beispielhaft und vorzugsweise Cholestyramin, Colestipol, Colesolvam, CholestaGel oder Colestimide, verabreicht.

Bei einer bevorzugten Ausführungsform der Erfindung wird die Verbindung der Formel (I) in Kombination mit einem Lipoprotein(a)-Antagonisten, wie beispielhaft und vorzugsweise Gemcabene calcium (CI-1027) oder Nicotinsäure, verabreicht.

Bei einer bevorzugten Ausführungsform der Erfindung wird die Verbindung der Formel (I) in Kombination mit einem Antagonisten des Niacin-Rezeptors, wie beispielhaft und vorzugsweise Niaspan, Acipimox oder Niceritrol, verabreicht.

Bei einer bevorzugten Ausführungsform der Erfindung wird die Verbindung der Formel (I) in Kombination mit einem Antioxidans, wie beispielhaft und vorzugsweise Probucol, AGI 1067 oder Bo 653, verabreicht.

Bei einer bevorzugten Ausführungsform der Erfindung wird die Verbindung der Formel (I) in Kombination mit einem LDL-Rezeptor-Inducer, wie beispielhaft Lifibrol, verabreicht.

Bei einer bevorzugten Ausführungsform der Erfindung wird die Verbindung der Formel (I) in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Ein weiterer Gegenstand der vorliegenden Erfindung sind Kombinationen der Verbindung der Formel (I) mit Substanzen, die eine Senkung der HMG-CoA-Reduktase-Genexpression bewirken. Derartige Substanzen können beispielsweise Inhibitoren der HMG-CoA-Reduktasetranskription oder der HMG-CoA-Reduktasetranslation sein. Eine Inhibition der HMG-CoA-Reduktase-Genexpression kann beispielsweise durch Inhibition der S1P (Site-1)-Protease oder durch Senkung der SREBP (sterol receptor binding protein)-Spiegel hervorgerufen werden.

Ein weiterer Gegenstand der vorliegenden Erfindung sind Kombinationen der Verbindung der Formel (I) mit Substanzen, die antientzündlich und/oder den arteriosklerotischen Plaque stabilisierend wirken. Derartige Substanzen können beispielsweise Wirkstoffe aus der Klasse der NSAIDs, der PAF-AH-Antagonisten oder der Chemokin-Rezeptor-Antagonisten, wie beispielhaft IL-8-Rezeptor-Antagonisten oder MCP-1-Antagonisten, darstellen.

Die erfindungsgemäßen Wirkstoffkombinationen besitzen wertvolle pharmakologische Eigenschaften und können zur Vorbeugung und Behandlung von Erkrankungen verwendet werden.

Die erfindungsgemäßen Wirkstoffkombinationen sind insbesondere zur Behandlung und zur primären oder sekundären Prävention koronarer Herzerkrankungen, z.B. von Myokardinfarkt, einsetzbar. Sie können zudem zur Behandlung und Prävention von Arteriosklerose, Restenose, Schlaganfällen sowie der Alzheimer'schen Krankheit verwendet werden. Darüber hinaus können die genannten Wirkstoffkombinationen auch zur Behandlung und Prävention von Hypolipoproteinämien, Dyslipidämien, Hypertriglyceridämien, Hyperlipidämien, Hypercholesterolämien, Fettsucht (Adipositas), Fettleibigkeit (Obesitas), Pankreatitis, Insulin-abhängigem und nicht-Insulin-abhängigem Diabetes, diabetischen Spätfolgen, wie beispielsweise Retinopathie, Nephropathie und Neuropathie, von kombinierten Hyperlipidämien sowie des Metabolischen Syndroms eingesetzt werden. Ferner eignen sich die erfindungsgemäßen Wirkstoffkombinationen zur Behandlung von Bluthochdruck, Herzinsuffizienz, Angina Pectoris, Ischämien sowie von entzündlichen Erkrankungen.

Weiterer Gegenstand der vorliegenden Erfindung sind Arzneimittel, die die erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäße Verbindung kann systemisch und/oder lokal wirken. Zu diesem Zweck kann sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege kann die erfindungsgemäße Verbindung in geeigneten Applikationsformen verabreicht werden.

Für die orale Applikation eignen sich nach dem Stand der Technik funktionierende, die erfindungsgemäße Verbindung schnell und/oder modifiziert abgebende Applikationsformen, die die erfindungsgemäße Verbindung in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäße Verbindung kann in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- CE: Cholesterinester
- CETP: Cholesterinester-Transfeiprotein
- DAST: Dimethylaminoschwefeltrifluorid
- DCI: direkte chemische Ionisation (bei MS)
- DDQ: 2,3-Dichlor-5,6-dicyano-1,4-benzochinon
- de: Diastereomerenüberschuss
- DMF: *N*,*N*-Dimethylformamid
- DMSO: Dimethylsulfoxid.
- d. Th.: der Theorie (bei Ausbeute)
- EDTA: Ethylendiamin-*N*,*N*,*N'*,*N'*-tetraessigsäure
- ee: Enantiomerenüberschuss
- eq.: Äquivalent(e)
- ESI: Elektrospray-Ionisation (bei MS)
- h: Stunde(n)
- HDL: High Density Lipoprotein
- HPLC: Hochdruck-, Hochleistungsflüssigchromatographie
- LC/MS: Flüssigchromatographie-gekoppelte Massenspektroskopie
- LDL: Low Density Lipoprotein
- min: Minute(n)
- MS: Massenspektroskopie
- NMR: Kernresonanzspektroskopie
- Rₜ: Retentionszeit (bei HPLC)
- SPA: Scintillation Proximity Assay
- TBAF: Tetrabutylammoniumfluorid
- TBDMSOTf: *tert.*-Butyldimethylsilyl-trifluormethansulfonat
- TFA: Trifluoressigsäure
- THF: Tetrahydrofuran

### HPLC- und LC/MS-Methoden:

Methode 1: Säule: Chiralpak IA, 250 mm x 4.6 mm; Eluent: Isohexan/1-Propanol 97:3; Fluss: 1.0 ml/min; UV-Detektion: 254 nm.

Methode 2: Instrument: HP 1100 mit DAD-Detektion; Säule: Kromasil RP-18, 60 mm x 2 mm, 3.5 µm; Eluent A: 5 ml HClO₄/l Wasser, Eluent B: Acetonitril; Gradient: 0 min 2% B → 0.5 min 2% B → 4.5 min 90% B → 9 min 90% B; Fluss: 0.75 ml/min; Temperatur: 30°C; UV-Detektion: 210 nm.

Methode 3 (LC/MS): Gerätetyp MS: Micromaß ZQ; Gerätetyp HPLC: HP 1100 Series; UV DAD; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Ausgansgsverbindungen und Intermediate: (a)

### Beispiel 1A

2-Cyclopentyl-4-cyclohexyl-7,7-dimethyl-3-(4-trifluormethylbenzoyl)-4,6,7,8-tetrahydro-1H-chinolin-5-on 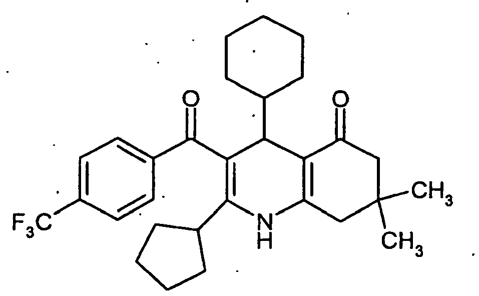 15.0 g (53 mmol, 1.2 eq.) 3-Amino-3-cyclopentyl-1-(4-trifluormethylphenyl)-propenon (Darstellung gemäß WO 03/028727, Beispiel 4) werden in 500 ml Diisopropylether vorgelegt und mit 6.80 ml (88 mmol, 2.0 eq.) Trifluoressigsäure und 6.19 g (44 mmol, 1 eq.) 5,5-Dimethylcyclohexan-1,3-dion versetzt. Nach Rühren bei Raumtemperatur für 10 min werden 7.1 ml (66 mmol, 1.5 eq.) Cyclohexancarbaldehyd zugegeben. Die Mischung wird anschließend für 15 h am Wasserabscheider unter Rückfluss erhitzt. Nach Abkühlen wird für 30 min im Eisbad gerührt. Der erhaltene Niederschlag wird abgesaugt und mit kaltem Diisopropylether gewaschen.

Ausbeute: 3.13 g (14% d. Th.) ¹H-NMR (CDCl₃, 300 MHz): δ = 0.77-2.05 (m, 20H), 1.17 (s, 6H), 2.21 (m, 2H), 2.40 (2d, 2H), 3.48 (sept, 1H), 3.79 (d, 1H), 5.85 (s, 1H), 7.66 (d, 2H), 7.78 (d, 2H) ppm.

MS (ESIpos): m/z = 500 [M+H]⁺.

### Beispiel 2A

2-Cyclopentyl-4-cyclohexyl-7,7-dimethyl-3-(4-trifluormethylbenzoyl)-7,8-dihydro-6*H*-chinolin-5-on 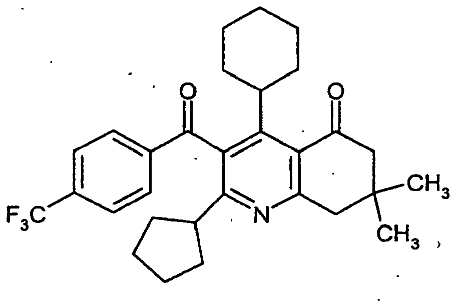 3.13 g (6.3 mmol) der Verbindung aus Beispiel 1A werden in 64 ml Dichlormethan gelöst und bei 0°C portionsweise mit 1.42 g (6.3 mmol) 2,3-Dichlor-5,6-dicyano-1,4-benzochinon (DDQ) versetzt. Es wird unter Rühren innerhalb von 3 h auf Raumtemperatur erwärmt. Die Mischung wird am Rotationsverdampfer eingeengt und der Rückstand durch Chromatographie gereinigt (Kieselgel, Laufmittel: Cyclohexan/Essigsäureethylester 5:1).

Ausbeute: 3.07 g (98.6% d. Th.)

¹H-NMR (CDCl₃, 400 MHz): δ = 1.01-1.22 (m, 2H), 1.10 (s, 3H), 1.18 (s, 3H), 1.35-2.00 (m, 16H), 2.50-2.69 (m, 3H), 3.07 (s, 2H), 3.35 (m, 1H), 7.75 (d, 2H), 7.94 (m, 2H) ppm.

MS (ESIpos): m/z = 498 [M+H]⁺.

### Beispiel 3A

[(5S)-2-Cyclopentyl-4-cyclohexyl-5-hydroxy-7,7-dimethyl-5,6,7,8-tetrahydrochinolin-3-yl](4-trifluormethylphenyl)-methanon 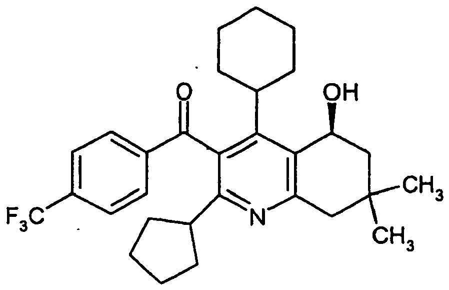 178 mg (1.2 mmol, 0.08 eq.) (*1R*,*2S*)-1-Aminoindan-2-ol werden in 400 ml THF vorgelegt und bei Raumtemperatur mit 9.73 g (60 mmol, 4 eq.) Boran-*N*,*N*-Diethylanilin-Komplex versetzt. Nach beendeter Gasentwicklung wird auf 0°C gekühlt, und 7.42 g (14.9 mmol, 1 eq.) der Verbindung aus Beispiel 2A, gelöst in 400 ml THF, werden zugegeben. Man lässt unter Rühren innerhalb von 16 h auf Raumtemperatur kommen. Nach erfolgter Umsetzung wird die Reaktionsmischung mit 20 ml Methanol versetzt, eingeengt und der Rückstand durch Chromatographie gereinigt (Kieselgel, Laufmittel: Isohexan/Essigsäureethylester-Gradient).

Ausbeute: 6.97 g (93.5% d. Th.)

Der Enantiomerenüberschuss wird nach Methode 1 zu 97.6% ee bestimmt.

¹H-NMR (CDCl₃, 400 MHz): δ = 0.90-2.10 (m, 27H), 2.55 (m, 1H), 2.70 (m, 1H), 2.80-3.40 (m, 2H), 5.18 (br. s, 1H), 6.8 (m, 2H), 7.40-8.40 (br. m, 4H) ppm.

MS (DCl/NH₃): m/z = 500 [M+H]⁺.

### Beispiel 4A

((*5S*)-5-{[*tert*.-Butyl(dimethyl)silyl]oxy}-4-cyclohexyl-2-cyclopentyl-7,7-dimethyl-5,6,7,8-tetrahydrochinolin-3-yl)[4-(trifluormethyl)phenyl]-methanon 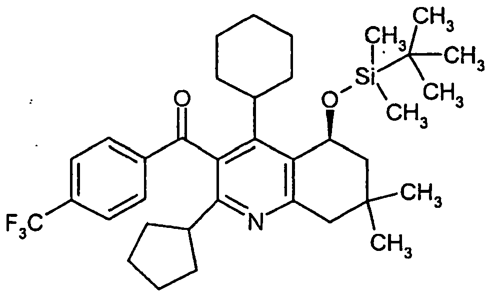 6.0 g (12.0 mmol) der Verbindung aus Beispiel 3A werden unter Argon in 45 ml trockenem Toluol vorgelegt. Anschließend werden 5.15 g (48.0 mmol, 4 eq.) 2,6-Lutidin bei Raumtemperatur zugegeben und das Gemisch auf -16°C gekühlt. Zu dieser Lösung werden 6.35 g (24.0 mmol, 2 eq.) Trifluormethansulfonsäure-*tert*.-butyldimethylsilylester in 15 ml Toluol tropfenweise hinzugefügt. Nach 15 min wird auf 0°C erwärmt und das Reaktionsgemisch 80 min bei dieser Temperatur gerührt. Zur Aufarbeitung wird 0.1 N Salzsäure (186 ml) hinzugegeben und nach Erwärmung auf Raumtemperatur mit Essigsäureethylester extrahiert. Die wässrige Phase wird noch dreimal mit Essigsäureethylester nachextrahiert, die vereinten organischen Phasen mit gesättigter Natriumhydrogencarbonat-Lösung und mit gesättigter Kochsalzlösung gewaschen und diese wässrige Phase wiederum mit Essigsäureethylester rückextrahiert. Die vereinten organischen Phasen werden über Natriumsulfat getrocknet, filtriert, im Vakuum eingeengt und der Rückstand durch Chromatographie gereinigt (Kieselgel, Laufmittel: Isohexan/Essigsäureethylester-Gradient).

Ausbeute: 5.96 g (80.8% d. Th.)

¹H-NMR (CDCl₃, 300 MHz): δ = 0.13 (s, 3H), 0.19 (s, 3H), 0.86 (s, 9H), 0.99-2.08 (m, 26H), 2.43-2.69 (m, 2H), 2.92-3.29 (m, 2H), 5.24 (br. s, 1H), 6.8 (m, 2H), 7.48-8.03 (br. m, 4H) ppm.

MS (DCI/NH₃): m/z = 614 [M+H]⁺.

### Beispiel 5A

(*S*)-((*5S*)-5-{[*tert*.-Butyl(dimethyl)silyl]oxy}-4-cyclohexyl-2-cyclopentyl-7,7-dimethyl-5,6,7,8-tetrahydrochinolin-3-yl)[4-(trifluormethyl)phenyl]methanol

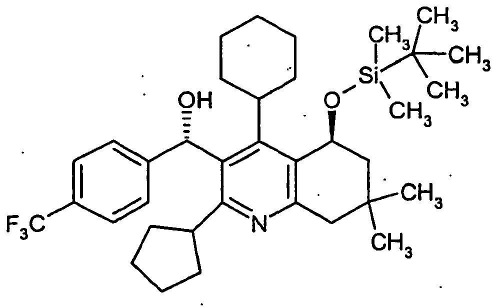

Zu einer Lösung von 5.72 g (9.3 mmol) der Verbindung aus Beispiel 4A in 116 ml trockenem THF werden bei 0°C 10.25 ml einer 1 M Lösung von Lithiumaluminiumhydrid (10.25 mmol, 1.1 eq.) in THF getropft. Es wird unter Rühren innerhalb von 6 h auf Raumtemperatur erwärmt. Zur Aufarbeitung werden 120 ml einer gesättigten Natrium-Kaliumtartrat-Lösung vorsichtig hinzugefügt. Nach Abklingen der Gasentwicklung wird dreimal mit Essigsäureethylester extrahiert, die vereinten organischen Phasen mit einer 1:1-Mischung aus Natriumhydrogencarbonat-Lösung und gesättigter Kochsalz-Lösung gewaschen und diese wässrige Phase wiederum mit Essigsäureethylester rückextrahiert. Die vereinten organischen Phasen werden über Natriumsulfat getrocknet, filtriert und im Vakuum eingeengt. Der Rückstand wird chromatographisch aufgereinigt und dabei die Produkt-Diastereomere aufgetrennt (Säule: Chiralpak AD, 500 mm x 40 mm, 20 µm; Eluent: Isohexan/Isopropanol 97.5:2.5; Fluss: 50 ml/min).

Ausbeute: 2.5 g (43.6% d. Th.)

Diastereomerenreinheit: 96.9% de, Rₜ = 4.15 min (Methode 1).

¹H-NMR (CDCl₃, 400 MHz): δ = 0.18 (br. s, 6H), 0.90 (s, 9H), 1.03-2.09 (m, 26H), 2.10-2.33 (br. m, 1H), 2.37-3.06 (m, 3H), 3.33 (m, 1H), 5.24 und 5.47 (2 br. s, 1H), 6.49 und 6.64 (2 br. s, 1H), 7.31-7.64 (m, 4H) ppm.

MS (ESIpos): m/z = 616 [M+H]⁺.

### syn-Diastereomer:

(*R*)-((*5S*)-5-{[*tert*.-Butyl(dimethyl)silyl]oxy}-4-cyclohexyl-2-cyclopentyl-7,7-dimethyl-5,6,7,8-tetrahydrochinolin-3-yl)[4-(trifluormethyl)phenyl]methanol

Ausbeute: 3.56 g (61.2% d. Th.)

Diastereomerenreinheit: 98.6% de, Rₜ = 2.77 min (Methode 1).

### Beispiel 6A

(*5S*)-5-{[*tert.*-Butyl(dimethyl)silyl]oxy}-4-cyclohexyl-2-cyclopentyl-3-{(*S*)-fluor[4-(trifluormethyl)phenyl]methyl}-7,7-dimethyl-5,6,7,8-tetrahydrochinolin

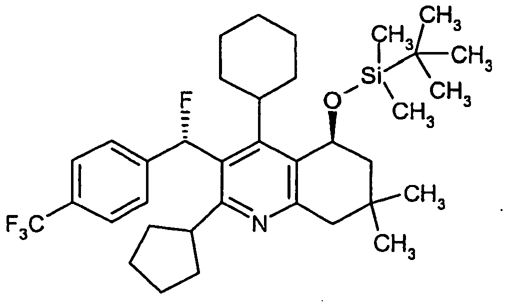

Zu einer Lösung von 9.96 g (16.2 mmol) der Verbindung aus Beispiel 5A in 300 ml trockenem Toluol werden bei -55°C unter Argon 3.21 ml Diethylaminoschwefeltrifluorid (24.3 mmol, 1.5 eq.) getropft. Es wird unter Rühren 1 h bei dieser Temperatur und anschließend weitere 2 h bei Raumtemperatur nachgerührt. Zur Aufarbeitung werden 120 ml einer gesättigten Natriumhydrogencarbonat-Lösung vorsichtig hinzugefügt. Es wird insgesamt dreimal mit Essigsäureethylester extrahiert. Die vereinten organischen Phasen werden mit gesättigter Kochsalz-Lösung gewaschen, über Natriumsulfat getrocknet, filtriert und im Vakuum eingeengt. Das Rohprodukt wird durch Filtration durch Kieselgel (Laufmittel: Cyclohexan/Essigsäureethylester 9:1) gereinigt.

Ausbeute: 9.93 g (99.3% d. Th.)

¹H-NMR (CDCl₃, 400 MHz): δ = 0.20 (br. s, 6H), 0.91 (s, 9H), 1.03-2.14 (m, 26H), 2.42-3.04 (m, 3H), 3.35 (m, 1H), 5.26 und 5.52 (2 br. s, 1H), 7.10-7.50 (m, 3H), 7.62 (d, 2H) ppm.

MS (ESIpos): m/z = 618 [M+H]⁺.

### Ausführungsbeispiele: (a)

### Beispiel 1

(*5S*)-4-Cyclohexyl-2-cyclopentyl-3-{(*S*)-fluor[4-(trifluormethyl)phenyl]methyl}-7,7-dimethyl-5,6,7,8-tetrahydrochinolin-5-ol

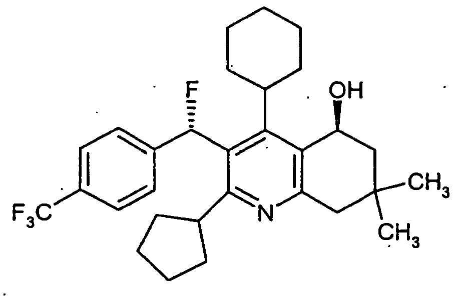

Zu einer Lösung von 9.91 g (16.0 mmol) der Verbindung aus Beispiel 6A in 200 ml trockenem THF werden bei 0°C 40.1 ml einer 1 M Lösung von Tetrabutylammoniumfluorid (40.1 mmol, 2.5 eq.) in THF getropft. Es wird auf Raumtemperatur erwärmt und 16 h lang bei dieser Temperatur nachgerührt. Zur Aufarbeitung wird mit 100 ml Essigsäureethylester verdünnt und zweimal mit je 100 ml Wasser sowie mit 50 ml gesättigter Kochsalz-Lösung gewaschen. Die organische Phase wird über Natriumsulfat getrocknet, filtriert und im Vakuum eingeengt. Das Rohprodukt wird chromatographisch gereinigt (Kieselgel, Laufmittel: Isohexan/Essigsäureethylester 100:0 → 1:1).

Ausbeute: 7.7 g (95.3% d. Th.)

¹H-NMR (CDCl₃, 300 MHz): δ = 1.02 (s, 3H), 1.17 (s, 3H), 1.08-2.15 (m, 21H), 2.59-3.00 (m, 3H), 3.51 (m, 1H), 5.13 (m, 1H), 7.34 (d, 2H), 7.39 (d, 1H), 7.61 (d, 2H) ppm.

MS (DCI): m/z = 504 [M+H]⁺

HPLC (Methode 2): Rₜ = 5.20 min.

### B. Bewertung der pharmakologischen Wirksamkeit (a)

### B-I. CETP-Inhibitions-Testung

### B-I.1. Gewinnung von CETP

CETP wird aus humanem Plasma durch Differential-Zentrifugation und Säulenchromatographie in partiell gereinigter Form gewonnen und zum Test verwendet Dazu wird humanes Plasma mit NaBr auf eine Dichte von 1.21 g pro ml eingestellt und 18 h bei 50.000 Upm und 4°C zentrifugiert. Die Bodenfraktion (d >1.21 g/ml) wird auf eine Sephadex®-Phenyl-Sepharose 4B-Säule (Fa. Pharmacia) aufgetragen, mit 0.15 M NaCl / 0.001 M TrisHCl pH 7.4 gewaschen und anschließend mit destilliertem Wasser eluiert. Die CETP-aktiven Fraktionen werden gepoolt, gegen 50 mM Natriumacetat pH 4.5 dialysiert und auf eine CM-Sepharose^{®}-Säule (Fa. Pharmacia) aufgetragen. Mit einem linearen Gradienten (0-1 M NaCl) wird anschließend eluiert. Die gepoolten CETP-Fraktionen werden gegen 10 mM TrisHCl pH 7.4 dialysiert und anschließend durch Chromatographie über eine Mono Q^{®}-Säule (Fa. Pharmacia) weiter gereinigt.

### B-I.2. CETP-Fluoreszenztest

Messung der CETP-katalysierten Übertragung eines fluoreszierenden Cholesterinesters zwischen Liposomen [modifiziert nach der Vorschrift von Bisgaier et al., J. Lipid Res. 34, 1625 (1993)]:
Zur Herstellung der Donorliposomen wird 1 mg Cholesteryl-4,4-difluor-5,7-dimethyl-4-bora-3a,4a-diaza-s-indacen-3-dodecanoat (Cholesteryl BODIPY^{®} FL C₁₂, Fa. Molecular Probes) mit 5.35 mg Triolein und 6.67 mg Phosphatidylcholin am Ultraschallbad unter leichtem Erwärmen in 600 µl Dioxan gelöst und diese Lösung sehr langsam unter Ultrabeschallung zu 63 ml 50 mM TrisHCl / 150 mM NaCl / 2 mM EDTA-Puffer pH 7.3 bei Raumtemperatur gegeben. Die Suspension wird anschließend unter N₂-Atmosphäre 30 min im Branson-Ultraschallbad bei ca. 50 Watt beschallt, wobei die Temperatur auf ca. 20°C gehalten wird.

Die Akzeptorliposomen werden analog aus 86 mg Cholesteryloleat, 20 mg Triolein und 100 mg Phosphatidylcholin, in 1.2 ml Dioxan und 114 ml des obigen Puffers gelöst, durch 30-minütige Ultrabeschallung bei 50 Watt (20°C) gewonnen.

### B-I.2.1. CETP-Fluoreszenztest mit angereichertem CETP

Zur Testung wird ein Testmix bestehend aus 1 Teil obigen Puffers, 1 Teil Donorliposomen und 2 Teilen Akzeptorliposomen verwendet.
50 µl Testmix werden mit 48 µl angereicherter CETP-Fraktion (1-3 µg), gewonnen über hydrophobe Chromatographie aus Humanplasma, sowie 2 µl einer Lösung der zu untersuchenden Substanz in DMSO versetzt und 4 h bei 37°C inkubiert.

Die Veränderung der Fluoreszenz bei 485/535 nm ist ein Maß für den CE-Transfer; die Hemmung des Transfers im Vergleich zum Kontrollansatz ohne Substanz wird ermittelt.

| **Beispiel Nr.** | **IC₅₀ [nM] Fluoreszenztest** |
|---|---|
| 1 | 25 |

### B-I.2.2. CETP-Fluoreszenztest mit humanem Plasma

42 µl (86% v/v) humanes Plasma (Sigma P9523) werden mit 6 µl (12% v/v) Donorliposomen sowie 1 µl (2% v/v) einer Lösung der zu untersuchenden Substanz in DMSO versetzt und 24 h bei 37°C inkubiert.

Die Veränderung der Fluoreszenz bei 510/520 nm (Spaltbreite 2.5 nm) ist ein Maß für den CE-Transfer; die Hemmung des Transfers im Vergleich zum Kontrollansatz ohne Substanz wird ermittelt.

| **Beispiel Nr.** | **IC₅₀ [nM] Fluoreszenztest in humanem Plasma** |
|---|---|
| 1 | 50 |

### B-I.2.3. Ex vivo-CETP-Fluoreszenztest

80 µl Testmix werden mit 10 µl Puffer sowie 2 µl Serum versetzt und 4 h bei 37°C inkubiert.

Die Veränderung der Fluoreszenz bei 485/535 nm ist ein Maß für den CE-Transfer; die Hemmung des Transfers im Vergleich zum Kontrollansatz ohne Substanz wird ermittelt.

### B-I.3. Gewinnung von radioaktiv markiertem HDL

50 ml frisches humanes EDTA-Plasma wird mit NaBr auf eine Dichte von 1.12 eingestellt und bei 4°C im Ty 65-Rotor 18 h bei 50.000 Upm zentrifugiert. Die Oberphase wird zur Gewinnung von kaltem LDL verwendet. Die Unterphase wird gegen 3 x 4 Liter PDB-Puffer (10 mM TrisHCl pH 7.4, 0.15 mM NaCl, 1 mM EDTA, 0.02% NaN₃) dialysiert. Pro 10 ml Retentatvolumen werden anschließend 20 µl ³H-Cholesterin (Dupont NET-725; 1 µC/µl gelöst in Ethanol) hinzugesetzt und 72 h bei 37°C unter N₂ inkubiert.

Der Ansatz wird dann mit NaBr auf die Dichte 1.21 eingestellt und im Ty 65-Rotor 18 h bei 50.000 Upm und 20°C zentrifugiert. Man gewinnt die Oberphase und reinigt die Lipoproteinfraktionen durch Gradientenzentrifugation. Dazu wird die isolierte, markierte Lipoproteinfraktion mit NaBr auf eine Dichte von 1.26 eingestellt. Je 4 ml dieser Lösung werden in Zentrifugenröhrchen (SW 40-Rotor) mit 4 ml einer Lösung der Dichte 1.21 sowie 4.5 ml einer Lösung der Dichte 1.063 überschichtet (Dichtelösungen aus PDB-Puffer und NaBr) und anschließend 24 h bei 38.000 Upm und 20°C im SW 40-Rotor zentrifugiert. Die zwischen der Dichte 1.063 und 1.21 liegende, das markierte HDL enthaltende Zwischenschicht wird gegen 3 x 100 Volumen PDB-Puffer bei 4°C dialysiert.

Das Retentat enthält radioaktiv markiertes ³H-CE-HDL, das auf ca. 5 x 106 cmp pro ml eingestellt zum Test verwendet wird.

### B-I.4. CETP-SPA-Test

Zur Testung der CETP-Aktivität wird die Übertragung von ³H-Cholesterolester von humanen HD-Lipoproteinen auf biotinylierte LD-Lipoproteine gemessen. Die Reaktion wird durch Zugabe von Streptavidin-SPA^{®}-beads (Fa. Amersham) beendet und die übertragene Radioaktivität direkt im Liquid Scintillation Counter bestimmt.

Im Testansatz werden 10 µl HDL-³H-Cholesterolester (ca. 50.000 cpm) mit 10 µl Biotin-LDL (Fa. Amersham) in 50 mM Hepes / 0.15 M NaCl / 0.1% Rinderserumalbumin (RSA) / 0.05% NaN₃ pH 7.4 mit 10 µl CETP (1 mg/ml) und 3 µl einer Lösung der zu prüfenden Substanz in 10% DMSO / 1% RSA für 18 h bei 37°C inkubiert. Anschließend werden 200 µl der SPA-Streptavidin-bead-Lösung (TRKQ 7005) zugesetzt, 1 h unter Schütteln weiter inkubiert und dann im Scintillationszähler gemessen. Als Kontrollen dienen entsprechende Inkubationen mit 10 µl Puffer, 10 µl CETP bei 4°C sowie 10 µl CETP bei 37°C.

Die in den Kontrollansätzen mit CETP bei 37°C übertragene Aktivität wird als 100% Übertragung gewertet. Die Substanzkonzentration, bei der diese Übertragung auf die Hälfte reduziert ist, wird, als IC₅₀-Wert angegeben.

| **Beispiel Nr.** | **IC₅₀ [nM] SPA-Test** |
|---|---|
| 1 | 7 |

### B-II.1. Messung der ex vivo-Aktivität an transgenen hCETP-Mäusen

Zur Prüfung auf CETP-inhibitorische Aktivität werden die Substanzen transgenen hCETP-Mäusen aus eigener Zucht [Dinchuk et al., BBA, 1295-1301 (1995)] oral mit der Schlundsonde verabreicht. Dazu werden männlich Tiere einen Tag vor Versuchsbeginn randomisiert Gruppen mit gleicher Tierzahl, in der Regel n = 4, zugeordnet. Vor der Substanzapplikation wird jeder Maus zur Bestimmung ihrer basalen CETP-Aktivität im Serum Blut durch Punktion des retroorbitalen Venenplexus entnommen (Zeitpunkt T1). Anschließend wird den Tieren die Testsubstanz mit der Schlundsonde verabreicht. Zu bestimmten Zeiten nach Applikation der Testsubstanz wird den Tieren ein zweites Mal Blut durch Punktion entnommen (Zeitpunkt T2), in der Regel 16 oder 24 h nach Substanzapplikation; gegebenenfalls kann dies aber auch zu einem anderen Zeitpunkt erfolgen.

Um die Hemmaktivität einer Substanz bewerten zu können, wird für jeden Zeitpunkt, also 16 bzw. 24 Stunden, eine entsprechende Kontrollgruppe eingesetzt, deren Tiere nur das Formulierungsmittel ohne Substanz erhalten. Bei den Kontrolltieren erfolgen die zwei Blutentnahmen pro Tier wie bei den substanzbehandelten Tieren, um die Veränderung der CETP-Aktivität ohne Inhibitor über den entsprechenden Versuchszeitraum (16 bzw. 24 h) bestimmen zu können.

Die Blutproben werden nach Abschluss der Gerinnung zentrifugiert und das Serum wird abpipettiert. Zur Bestimmung der CETP-Aktivität wird der Cholesterylester-Transport über 4 h bestimmt. Dazu werden im Testansatz in der Regel 2 µl Serum eingesetzt; der Test wird wie unter B-I.2.3. beschrieben durchgeführt.

Die Differenzen im Cholesterylester-Transport [pM CE/h (T2) - pM CE/h (T1)] werden für jedes Tier berechnet und in den Gruppen gemittelt. Eine Substanz, die zu einem der Zeitpunkte den Cholesterylester-Transport um >20% herabsetzt, wird als wirksam angesehen.

| **Beispiel Nr.** | **% Hemmung bei 3 mg/kg** | |
|---|---|---|
| | 16h | 24 h |
| 1 | 50 | 24 |

### B-II.2. Messung der in vivo-Wirksamkeit an Syrischen Goldhamstern

Zur Bestimmung der oralen Wirkung von CETP-Inhibitoren auf Serum-Lipoproteine und -Triglyceride werden 150-200 g schwere weibliche syrische Goldhamster aus eigener Zucht (Stamm BAY:DSN) verwendet. Die Tiere werden pro Käfig zu sechst gruppiert und zwei Wochen bei Futter und Wasser *ad libitum* akklimatisiert.

Unmittelbar vor Versuchsbeginn und nach Abschluss der Substanzapplikation wird mittels retroorbitaler Punktion des Venenplexus Blut entnommen, woraus nach 30 min Inkubation bei Raumtemperatur und 20 min Zentrifugation bei 30.000 g Serum gewonnen wird. Die Substanzen werden in 20% Solutol / 80% Wasser gelöst und mit Hilfe einer Schlundsonde peroral verabreicht. Die Kontrolltiere erhalten identische Volumina Lösungsmittel ohne Testsubstanz.

Die Bestimmung von Triglyceriden, Gesamt-Cholesterin, HDL-Cholesterin und LDL-Cholesterin erfolgt mit Hilfe des Analysegeräts COBAS INTEGRA 400 plus (Fa. Roche Diagnostics) nach Angaben des Herstellers. Aus den Messwerten wird für jeden Parameter die durch Substanzbehandlung hervorgerufene prozentuale Veränderung für jedes Tier berechnet und als Mittelwert mit Standardabweichung pro Gruppe (n = 6 oder n = 12) angegeben. Sind die Substanzeffekte im Vergleich zur Lösungsmittel-behandelten Gruppe signifikant, wird der mittels t-Test ermittelte p-Wert hinzugefügt (* p ≤0.05; ** p ≤0.01; *** p ≤0.005).

| **Beispiel Nr.** | **% HDL-Anstieg nach 24 h (Dosis: 2 x 10 mg/kg)** |
|---|---|
| 1 | 23 |

### B-II.3. Messung der in vivo-Wirksamkeit an transgenen hCETP-Mäusen

Zur Bestimmung der oralen Wirkung auf Lipoproteine und Triglyceride wird transgenen Mäusen [Dinchuk et al., BBA, 1295-1301 (1995)] Testsubstanz mit der Schlundsonde verabreicht. Vor Versuchsbeginn wird den Mäusen retroorbital Blut entnommen, um Cholesterin und Triglyceride im Serum zu bestimmen. Das Serum wird, wie oben für Hamster beschrieben, durch Inkubation bei 4°C über Nacht und anschließende Zentrifugation bei 6.000 g gewonnen. Nach drei Tagen wird den Mäusen wieder Blut entnommen, um Lipoproteine und Triglyceride erneut zu bestimmen. Die Veränderungen der gemessenen Parameter werden als prozentuale Veränderung gegenüber dem Ausgangswert ausgedrückt.

| **Beispiel Nr.** | **% HDL-Anstieg nach 3 d (Dosis: 3 x 3 mg/kg)** |
|---|---|
| 1 | 91 |

### C. Ausführungsbeispiele für pharmazeutische Zusammensetzungen (a)

Die erfindungsgemäße Verbindung kann folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Verstellung:

Das Rhodigel wird in Ethanol suspendiert, die erfindungsgemäße Verbindung wird der Suspension zugefügt. Unter Rühren erfolgt die Zugabe des Wassers. Bis zum Abschluss der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

### Ausgangsverbindungen und Intermediate: (b)

### Beispiel 1A

4-cyclopentyl-2-isopropyl-7,7-dimethyl-3-[4-(tifluormethyl)benzoyl]-4,6,7,8-tetahydrochinolin-5(*1H*)-on 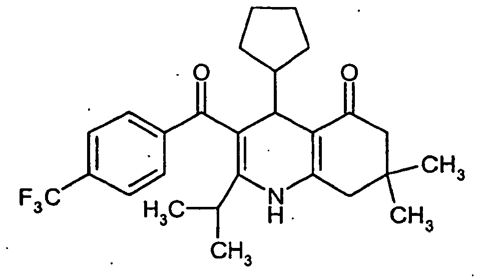 10.0 g (38.9 mmol, 1.0 eq.) 3-Amino-3-isopropyl-1-(4-trifluormethylphenyl)-propenon (Darstellung gemäß WO 03/028727, Beispiel 2) werden in 300 ml Diisopropylether vorgelegt und mit 2.99 ml (38.9 mmol, 1.0 eq.) Trifluoressigsäure und 5.45 g (38.9 mmol, 1 eq.) 5,5-Dimethylcyclohexan-1,3-dion versetzt. Nach Rühren bei Raumtemperatur für 10 min wird zum Rückfluss erhitzt und 4.58 g (46.7 mmol, 1.2 eq.) Cyclopentancarbaldehyd zugegeben. Die Mischung wird für 15 h am Wasserabscheider unter Rückfluss erhitzt. Nach Abkühlen wird für 45 min im Eisbad gerührt, der erhaltene Niederschlag abgesaugt, mit kaltem Diisopropylether gewaschen und im Hochvakuum von Lösungsmittelresten befreit.

Ausbeute: 4.15 g (23% d. Th.)

¹H-NMR (CDCl₃, 400 MHz): δ = 1.05 (d, 3H), 1.15 (s, 3H), 1.16 (s, 3H), 1.28 (d, 3H), 1.24-1.61 (m, 7H), 2.30 und 2.51 (2d, 2H), 2.34 (s, 2H), 3.49 (sept, 1H), 3.81 (d, 1H), 5.96 (s, 1H), 7.66 (d, 2H), 7.77 (d, 2H) ppm.

MS (DCI/NH₃): m/z = 460 [M+H]⁺, 477 [M+NH₄]⁺.

### Beispiel 2A

4-Cyclopentyl-2-isopropyl-7,7-dimethyl-3-[4-(trifluormethyl)benzoyl]-7,8-dihydrochinolin-5(*6H*)-on 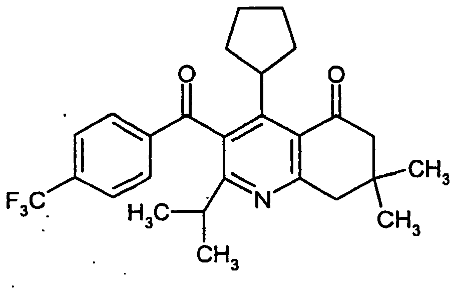 4.0 g (8.7 mmol) der Verbindung aus Beispiel 1A werden in 100 ml Dichlormethan gelöst und bei 0°C portionsweise mit 2.17 g (9.6 mmol, 1.1 eq.) 2,3-Dichlor-5,6-dicyano-1,4-benzochinon (DDQ) versetzt. Es wird unter Rühren innerhalb von 3 h auf Raumtemperatur erwärmt. Die Mischung wird am Rotationsverdampfer eingeengt und der Rückstand durch Chromatographie gereinigt (Kieselgel, Laufmittel: Isohexan/Essigsäureethylester 100:0 → 50:50).

Ausbeute: 3.78 g (95.1% d. Th.)

¹H-NMR (CDCl₃, 300 MHz): δ = 1.09 (d, 3H), 1.11 (s, 3H), 1.17 (s, 3H), 1.19 (d, 3H), 1.34-2.00 (m, 8H), 2.51-2.68 (m, 3H), 3.01 (m, 1H), 3.1 (s, 2H), 7.76 (d, 2H), 7.94 (m, 2H) ppm.

MS (ESIpos): m/z = 458 [M+H]⁺.

### Beispiel 3A

[(*5S*)-4-Cyclopentyl-5-hydroxy-2-isopropyl-7,7-dimethyl-5,6,7,8-tetrahydrochinolin-3-yl][4-(trifluormethyl)phenyl]methanon 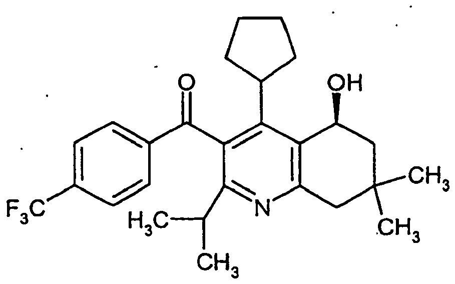 140 mg (0.96 mmol, 0.08 eq.) (*1R*,*2S*)-1-Aminoindan-2-ol werden in 440 ml THF vorgelegt und bei Raumtemperatur mit 7.80 g (47.8 mmol, 4.0 eq.) Boran-*N*,*N*-Diethylanilin-Komplex versetzt. Nach beendeter Gasentwicklung wird auf 0°C gekühlt, und 5.47 g (12 mmol, 1 eq.) der Verbindung aus Beispiel 2A, gelöst in 40 ml THF, werden zugegeben. Man lässt unter Rühren innerhalb von 28 h auf Raumtemperatur kommen. Nach erfolgter Umsetzung wird die Reaktionsmischung mit 20 ml Methanol versetzt und eingeengt. Der Rückstand wird zwischen 150 ml Wasser und 150 ml. Essigsäureethylester verteilt. Die wässrige Phase wird zweimal mit je 100 ml Essigsäureethylester extrahiert. Die vereinigten organischen Phasen werden mit 50 ml gesättigter Natriumchlorid-Lösung gewaschen, über Natriumsulfat getrocknet, filtriert und im Vakuum eingeengt. Anschließend wird das Rohprodukt durch Chromatographie gereinigt (Kieselgel, Laufmittel: Isohexan/Essigsäureethylester 4:1).

Ausbeute: 4.97 g (90.5% d. Th.)

Der Enantiomerenüberschuss wird nach Methode 1 zu 92.5% ee bestimmt.

Die Enantiomere werden durch Chromatographie an chiraler Phase getrennt (Säule: Chiralpak AD, 500 mm x 40 mm, 20 µm; Eluent: Isohexan/Isopropanol 97.5:2.5; Fluss: 50 ml/min):

Ausbeute: 4.46 g (81.2% d. Th.)

Der Enantiomerenüberschuss wird nach Methode 1 zu 98.1% ee bestimmt; Rₜ (Methode 1) = 7.09 min.

¹H-NMR (CDCl₃, 300 MHz): δ = 0.94-1.30 (m, 12H), 1.31-2.03 (m, 11H), 2.53 (m, 1H), 2.72 (d, 1H), 2.95-3.12 (m, 1H), 3.29 (m, 1H), 5.18 (m, 1H), 7.73 (d, 2H), 7.93 (m, 2H) ppm.

MS (DCI): m/z = 460 [M+H]⁺.

### Bespiel 4A

((*5S*)-5-{[*tert*.-Butyl(dimethyl)silyl]oxy}-4-cyclopentyl-2-isopropyl-7,7-dimethyl-5,6,7,8-tetrahydrochinolin-3-yl)[4-(trifluornlethyl)phenyl]methanon 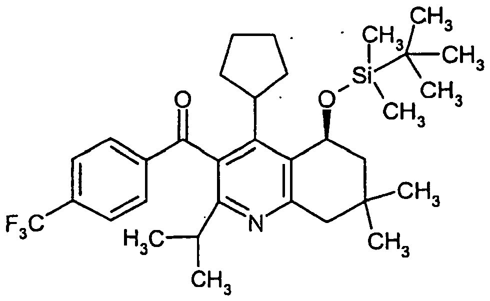 3.65 g (7.95 mmol) der Verbindung aus Beispiel 3A werden unter Argon in 80 ml trockenem Toluol vorgelegt Anschließend werden 3.41 g (31.8 mmol, 4 eq.) 2,6-Lutidin bei Raumtemperatur zugegeben und das Gemisch auf -18°C gekühlt. Zu dieser Lösung werden 3.65 ml (15.9 mmol, 2 eq.) Trifluormethansulfonsäure-*tert*.-butyldimethylsilylester tropfenweise hinzugefügt. Nach 20 min wird auf 0°C erwärmt und das Reaktionsgemisch weitere 55 min bei dieser Temperatur gerührt. Zur Aufarbeitung wird gesättigte Ammoniumchlorid-Lösung (100 ml) hinzugegeben und nach Erwärmung auf Raumtemperatur mit Essigsäureethylester extrahiert. Die wässrige Phase wird noch zweimal mit Essigsäureethylester extrahiert, die vereinigten organischen Phasen mit gesättigter Kochsalz-Lösung gewaschen, über Natriumsulfat getrocknet, filtriert und im Vakuum eingeengt. Der Rückstand wird durch Chromatographie gereinigt (Kieselgel, Laufmittel: Isohexan/Essigsäureethylester 9:1).

Ausbeute: 4.65 g (quantitativ)

¹H-NMR (CDCl₃, 400 MHz): δ = 0.09 (s, 3H), 0.18 (s, 3H), 0.86 (s, 9H), 0.92 (s, 3H), 1.04.(d, 3H), 1.22 (d, 3H), 1.24 (s, 3H), 1.27-1.86 (m, 9H), 1.93-2.02 (m, 1H), 2.50 (m, 1H), 2.58-3.23 (m, 2H), 3.32 (m, 1H), 5.20 (m, 1H), 7.73 (d, 2H), 7.83-8.00 (m, 2H) ppm.

MS (ESIpos): m/z = 574 [M+H]⁺.

### Beispiel 5A

*(S)-((5S)-5*-*{[tert.-*Butyl(dimethyl)silyl]oxy}-4-cyclopentyl-2-isopropyl-7,7-dimethyl-5,6,7,8-tetrahydrochinolin-3-yl)[4-(trifluormethyl)phenyl]methanol

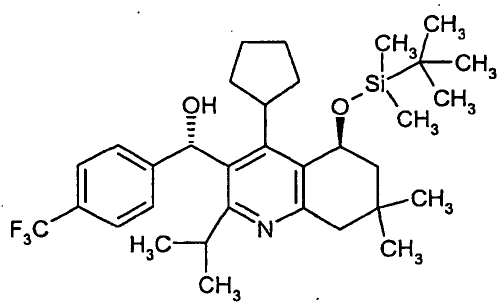

Zu einer Lösung von 4.59 g (8.0 mmol) der Verbindung aus Beispiel 4A in 80 ml trockenem THF werden bei 0°C 12.0 ml einer 1 M Lösung von Lithiumaluminiumhydrid (12.0 mmol, 1.5 eq.) in THF getropft. Es wird unter Rühren innerhalb von 16 h auf Raumtemperatur erwärmt. Zur Aufarbeitung werden 120 ml einer gesättigten Natrium-Kaliumtartrat-Lösung vorsichtig hinzugefügt. Nach Abklingen der Gasentwicklung wird zweimal mit Essigsäureethylester extrahiert, die vereinigten organischen Phasen mit gesättigter Kochsalz-Lösung gewaschen, über Natriumsulfat getrocknet, filtriert und im Vakuum eingeengt. Der Rückstand wird chromatographisch aufgereinigt und dabei die Produkt-Diastereomere aufgetrennt (Kieselgel, Laufmittel: Isohexan/Essigsäureethylester 95:5).

Ausbeute: 2.29 g (49.8% d. Th.)

¹H-NMR (CDCl₃, 300 MHz): δ = 0.13 (s, 3H), 0.20 (s, 3H), 0.64-1.00 (m, 18H), 1.09-2.23 (m, 14H), 2.59 (d, 1H), 2.89 (m, 1H), 3.00 (m, 1H), 3.71 (m, in), 5.21 (t, 1H), 6.22 (br. s, 1H), 7.43 (d, 2H), 7.59 (d, 2H) ppm.

MS (ESIpos): m/z = 576 [M+H]⁺

R_{f}= 0.26 (Isohexan/Essigsäureethylester 9:1).

### syn-Diastereomer:

(*R*)-((*5S*-5-([*tert*.-Butyl(dimethyl)silyl]oxy}-4-cyclopentyl-2-isopropyl-7,7-dimethyl-5,6,7,8-tetrahydrochinolin-3-yl)[4-(trifluormethyl)phenyl]methanol

Ausbeute: 2.48 g (53.9% d. Th.)

R_{f}= 0.34 (Isohexan/Essigsäureethylester 9:1).

### Beispiel 6A

(*5S*)-5-{[*tert*.-Butyl(dimethyl)silyl]oxy}-4-cyclopentyl-3-{(S)-fluor[4-(trifluorinethyl)phenyl]-methyl}-2-isopropyl-7,7-dimethyl-5,6,7,8-tetrahydrochinolin

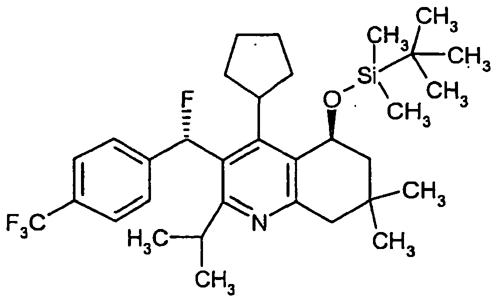

Zu einer Lösung von 2.38 g (4.1 mmol) der Verbindung aus Beispiel 5A in 40 ml trockenem Dichlormethan werden bei -10°C unter Argon 0.82 ml Diethylaminoschwefeltrifluorid (6.2 mmol, 1.5 eq.) getropft. Es wird 200 min bei dieser Temperatur nachgerührt. Zur Aufarbeitung werden unter Eiskühlung 40 ml einer gesättigten Natriumhydrogencarbonat-Lösung vorsichtig hinzugefügt. Es wird insgesamt dreimal mit Essigsäureethylester extrahiert. Anschließend werden die vereinten organischen Phasen mit gesättigter Kochsalz-Lösung gewaschen, über Natriumsulfat getrocknet, filtriert und im Vakuum eingeengt. Das Rohprodukt wird durch Filtration durch Kieselgel (Laufmittel: Cyclohexan/Essigsäureethylester 9: 1) gereinigt.

Ausbeute: 1.92 g (80.3% d. Th.)

LC/MS (Methode 3): Rₜ = 3.85 min.

MS (ESIpos): m/z = 578 [M+H]⁺

R_{f} = 0.66 (Isohexan/Essigsäureethylester 9:1).

### Ausführungsbeispiele: (b)

### Beispiel 1

(*5S*)-4-Cyclopentyl-3-{(*S*)-fluor[4-(trifluormethyl)phenyl]methyl)-2-isopropyl-7,7-dimethyl-5,6,7,8-tetrahydrochinolin-5-ol

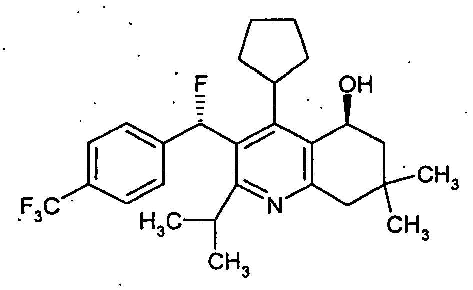

Zu einer Lösung von 1.92 g (3.3 mmol) der Verbindung aus Beispiel 6A in 20 ml trockenem THF werden bei 0°C 13.3 ml einer 1 M Lösung von Tetrabutylammoniumfluorid (13.3 mmol, 4.0 eq.) in THF getropft. Die Reaktionsmischung wird 4 h im Eisbad gerührt. Zur Aufarbeitung wird mit 100 ml Essigsäureethylester verdünnt und zweimal mit je 100 ml Wasser sowie mit 50 ml gesättigter Kochsalz-Lösung gewaschen. Die organische Phase wird über Natriumsulfat getrocknet, filtriert und im Vakuum eingeengt. Das Rohprodukt wird chromatographisch gereinigt (Kieselgel, Laufmittel: Isohexan/Essigsäureethylester 9:1 → 2: 1).

Ausbeute: 1.18 g (76.4% d. Th.) ¹H-NMR (CDCl₃, 300 MHz): δ = 0.70 (d, 3H), 1:03 (s, 3H), 1.13 (d, 3H), 1.19 (s, 3H), 1.32-2.20 (m, 11H), 2.63-2.97 (m, 3H), 3.86 (m, 1H), 5.15 (m, 1H), 6.94 (d, 1H), 7.34 (d, 2H), 7.61 (d, 2H) ppm.

MS (DCI): m/z = 464 [M+H]⁺

R_{f}= 0.13 (Isohexan/Essigsäureethylester 4:1).

Die weitere Abtrennung von im Produkt noch enthaltenen Diastereomeren erfolgt chromatographisch (Säule: Chiralpak AD, 500 mm x 40 mm, 20 µm; Eluent: Isohexan/Isopropanol 97.5:2.5; Fluss: 50 ml/min).

Ausbeute: 0.35 g (22.9% d. Th.).

### B. Bewertung der pharmakologischen Wirksamkeit (b)

### B-I. CETP-Inhibitions-Testung

### B-L1. Gewinnung von CETP

CETP wird aus humanem Plasma durch Differential-Zentrifugation und Säulenchromatographie in partiell gereinigter Form gewonnen und zum Test verwendet. Dazu wird humanes Plasma mit NaBr auf eine Dichte von 1.21 g pro ml eingestellt und 18 h bei 50.000 Upm und 4°C zentrifugiert. Die Bodenfraktion (d >1.21 g/ml) wird auf eine Sephadex^{®}-Phenyl-Sepharose 4B-Säule (Fa. Pharmacia) aufgetragen, mit 0.15 M NaCl / 0.001 M TrisHCl pH 7.4 gewaschen und anschließend mit destilliertem Wasser eluiert. Die CETP-aktiven Fraktionen werden gepoolt, gegen 50 mM Natriumacetat pH 4.5 dialysiert und auf eine CM-Sepharose^{®}-Säule (Fa. Pharmacia) aufgetragen. Mit einem linearen Gradienten (0-1 M NaCl) wird anschließend eluiert. Die gepoolten CETP-Fraktionen werden gegen 10 mM TrisHCl pH 7.4 dialysiert und anschließend durch Chromatographie über eine Mono Q^{⊗}-Säule (Fa. Pharmacia) weiter gereinigt.

### B-I.2. CETP-Fluoreszenztest

Messung der CETP-katalysierten Übertragung eines fluoreszierenden Cholesterinesters zwischen Liposomen [modifiziert nach der Vorschrift von Bisgaier et al., J. Lipid Res. 34, 1625 (1993)]:
Zur Herstellung der Donorliposomen wird 1 mg Cholesteryl-4,4-difluor-5,7-dimethyl-4-bora-3a,4a-diaza-s-indacen-3-dodecanoat (Cholesteryl BODIPY^{®} FL C₁₂, Fa. Molecular Probes) mit 5.35 mg Triolein und 6.67 mg Phosphatidylcholin am Ultraschallbad unter leichtem Erwärmen in 600 µl Dioxan gelöst und diese Lösung sehr langsam unter Ultrabeschallung zu 63 ml 50 mM TrisHCl / 150 mM NaCl/2 mM EDTA-Puffer pH 7.3 bei Raumtemperatur gegeben. Die Suspension wird anschließend unter N₂-Atmosphäre 30 min im Branson-Ultraschallbad bei ca. 50 Watt beschallt, wobei die Temperatur auf ca. 20°C gehalten wird.

Die Akzeptorliposomen werden analog aus 86 mg Cholesteryloleat, 20 mg Triolein und 100 mg Phosphatidylcholin, in 1.2 ml Dioxan und 114 ml des obigen Puffers gelöst, durch 30-minütige Ultrabeschallung bei 50 Watt (20°C) gewonnen.

### B-I.2.1. CETP-Fluoreszenztest mit angereichertem CETP

Zur Testung wird ein Testmix bestehend aus 1 Teil obigen Puffers, 1 Teil Donorliposomen und 2 Teilen Akzeptorliposomen verwendet.

50 µl Testmix werden mit 48 µl angereicherter CETP-Fraktion (1-3 µg), gewonnen über hydrophobe Chromatographie aus Humanplasma, sowie 2 µl einer Lösung der zu untersuchenden Substanz in DMSO versetzt und 4 h bei 37°C inkubiert.

Die Veränderung der Fluoreszenz bei 485/535 nm ist ein Maß für den CE-Transfer; die Hemmung des Transfers im Vergleich zum Kontrollansatz ohne Substanz wird ermittelt.

| **Beispiel Nr.** | **IC₅₀ [nM] Fluoreszenztest** |
|---|---|
| 1 | 25 |

### B-I.2.2. CETP-Fluoreszenztest mit humanem Plasma

42 µl (86% v/v) humanes Plasma (Sigma P9523) werden mit 6 µl (12% v/v) Donorliposomen sowie 1 µl (2% v/v) einer Lösung der zu untersuchenden Substanz in DMSO versetzt und 24 h bei 37°C inkubiert.

Die Veränderung der Fluoreszenz bei 510/520 nm (Spaltbreite 2.5 nm) ist ein Maß für den CE-Transfer; die Hemmung des Transfers im Vergleich zum Kontrollansatz ohne Substanz wird ermittelt.

| **Beispiel Nr.** | **ICso [nM] Fluoreszenztest in humanem Plasma** |
|---|---|
| 1 | 84 |

### B-I.2.3. Ex vivo-CETP-Fluoreszenztest

80 µl Testmix werden mit 10 µl Puffer sowie 2 µl Serum versetzt und 4 h bei 37°C inkubiert

Die Veränderung der Fluoreszenz bei 485/535 nm ist ein Maß für den CE-Transfer; die Hemmung des Transfers im Vergleich zum Kontrollansatz ohne Substanz wird ermittelt.

### B-I.3. Gewinnung von radioaktiv markiertem HDL

50 ml frisches humanes EDTA-Plasma wird mit NaBr auf eine Dichte von 1.12 eingestellt und bei 44°C im Ty 65-Rotor 18 h bei 50.000 Upm zentrifugiert. Die Oberphase wird zur Gewinnung von kaltem LDL verwendet. Die Unterphase wird gegen 3 x 4 Liter PDB-Puffer (10 mM TrisHCl pH 7.4, 0.15 mM NaCl, 1 mM EDTA, 0.02% NaN₃) dialysiert. Pro 10 ml Retentatvolumen werden anschließend 20 µl ³H-Cholesterin (Dupont NET-725; 1 µC/µl gelöst in Ethanol) hinzugesetzt und 72 h bei 37°C unter N₂ inkubiert.

Der Ansatz wird dann mit NaBr auf die Dichte 1.21 eingestellt und im Ty 65-Rotor 18 h bei 50.000 Upm und 20°C zentrifugiert. Man gewinnt die Oberphase und reinigt die Lipoproteinfraktionen durch Gradientenzentrifugation. Dazu wird die isolierte, markierte Lipoproteinfraktion mit NaBr auf eine Dichte von 1.26 eingestellt. Je 4 ml dieser Lösung werden in Zentrifugenröhrchen (SW 40-Rotor) mit 4 ml einer Lösung der Dichte 1.21 sowie 4.5 ml einer Lösung der Dichte 1.063 überschichtet (Dichtelösungen aus PDB-Puffer und NaBr) und anschließend 24 h bei 38.000 Upm und 20°C im SW 40-Rotor zentrifugiert. Die zwischen der Dichte 1.063 und 1.21 liegende, das markierte HDL enthaltende Zwischenschicht wird gegen 3 x 100 Volumen PDB-Puffer bei 4°C dialysiert.

Das Retentat enthält radioaktiv markiertes ³H-CE-HDL, das auf ca. 5 x 106 cmp pro ml eingestellt zum Test verwendet wird.

### B-I.4. CETP-SPA-Test

Zur Testung der CETP-Aktivität wird die Übertragung von ³H-Cholesterolester von humanen HD-Lipoproteinen auf biotinylierte LD-Lipoproteine gemessen. Die Reaktion wird durch Zugabe von Streptavidin-SPA^{⊗}-beads (Fa. Amersham) beendet und die übertragene Radioaktivität direkt im Liquid Scintillation Counter bestimmt.

Im Testansatz werden 10 µl HDL-³H-Cholesterolester (ca. 50.000 cpm) mit 10 µl Biotin-LDL (Fa. Amersham) in 50 mM Hepes / 0.15 M NaCl / 0.1% Rinderserumalbumin (RSA) / 0.05% NaN₃ pH 7.4 mit 10 µl CETP (1 mg/ml) und 3 µl einer Lösung der zu prüfenden Substanz in 10% DMSO / 1% RSA für 18 h bei 37°C inkubiert. Anschließend werden 200 µl der SPA-Streptavidin-bead-Lösung (TRKQ 7005) zugesetzt, 1 h unter Schütteln weiter inkubiert und dann im Scintillationszähler gemessen. Als Kontrollen dienen entsprechende Inkubationen mit 10 µl Puffer, 10 µl CETP bei 4°C sowie 10 µl CETP bei 37°C.

Die in den Kontrollansätzen mit CETP bei 37°C übertragene Aktivität wird als 100% Übertragung gewertet. Die Substanzkonzentration, bei der diese Übertragung auf die Hälfte reduziert ist, wird als IC_{5O}-Wert angegeben.

| **Beispiel Nr.** | **IC₅₀ [nM] SPA-Test** |
|---|---|
| 1 | 12 |

### B-II.1. Messung der ex vivo-Aktivität an transgenen hCETP-Mäusen

Zur Prüfung auf CETP-inhibitorische Aktivität werden die Substanzen transgenen hCETP-Mäusen aus eigener Zucht [Dinchuk et al., BBA, 1295-1301 (1995)] oral mit der Schlundsonde verabreicht. Dazu werden männliche Tiere einen Tag vor Versuchsbeginn randomisiert Gruppen mit gleicher . Tierzahl, in der Regel n = 4, zugeordnet. Vor der Substanzapplikation wird jeder Maus zur Bestimmung ihrer basalen CETP-Aktivität im Serum Blut durch Punktion des retroorbitalen Venenplexus entnommen (Zeitpunkt T1). Anschließend wird den Tieren die Testsubstanz mit der Schlundsonde verabreicht. Zu bestimmten Zeiten nach Applikation der Testsubstanz wird den Tieren ein zweites Mal Blut durch Punktion entnommen (Zeitpunkt T2), in der Regel 16 oder 24 h nach Substanzapplikation; gegebenenfalls kann dies aber auch zu einem anderen Zeitpunkt erfolgen.

Um die Hemmaktivität einer Substanz bewerten zu können, wird für jeden Zeitpunkt, also 16 bzw. 24 Stunden, eine entsprechende Kontrollgruppe eingesetzt, deren Tiere nur das Formulierungsmittel ohne Substanz erhalten. Bei den Kontrolltieren erfolgen die zwei Blutentnahmen pro Tier wie bei den substanzbehandelten Tieren, um die Veränderung der CETP-Aktivität ohne Inhibitor über den entsprechenden Versuchszeitraum (16 bzw. 24 h) bestimmen zu können.

Die Blutproben werden nach Abschluss der Gerinnung zentrifugiert und das Serum wird abpipettiert Zur Bestimmung der CETP-Aktivität wird der Cholesterylester-Transport über 4 h bestimmt. Dazu werden im Testansatz in der Regel 2 µl Serum eingesetzt; der Test wird wie unter B-I.2.3. beschrieben durchgeführt.

Die Differenzen im Cholesterylester-Transport [pM CE/h (T2) - pM CE/h (T1)] werden für jedes Tier berechnet und in den Gruppen gemittelt. Eine Substanz, die zu einem der Zeitpunkte den Cholesterylester-Transport um >20% herabsetzt, wird als wirksam angesehen.

| **Beispiel Nr.** | **% Hemmung bei 3 mg/kg** | |
|---|---|---|
| | 16h | 24 h |
| 1 | 49 | 39 |

### B-II.2. Messung der in vivo-Wirksamkeit an Syrischen Goldhamstern

Zur Bestimmung der oralen Wirkung von CETP-Inhibitoren auf Serum-Lipoproteine und -Triglyceride werden 150-200 g schwere weibliche syrische Goldhamster aus eigener Zucht (Stamm BAY:DSN) verwendet. Die Tiere werden pro Käfig zu sechst gruppiert und zwei Wochen bei Futter und Wasser *ad libitum* akklimatisiert.

Unmittelbar vor Versuchsbeginn und nach Abschluss der Substanzapplikation wird mittels retroorbitaler Punktion des Venenplexus Blut entnommen, woraus nach 30 min Inkubation bei Raumtemperatur und 20 min Zentrifugation bei 30.000 g Serum gewonnen wird. Die Substanzen werden in 20% Solutol / 80% Wasser gelöst und mit Hilfe einer Schlundsonde peroral verabreicht. Die Kontrolltiere erhalten identische Volumina Lösungsmittel ohne Testsubstanz.

Die Bestimmung von Triglyceriden, Gesamt-Cholesterin, HDL-Cholesterin und LDL-Cholesterin erfolgt mit Hilfe des Analysegeräts COBAS INTEGRA 400 plus (Fa. Roche Diagnostics) nach Angaben des Herstellers. Aus den Messwerten wird für jeden Parameter die durch Substanzbehandlung hervorgerufene prozentuale Veränderung für jedes Tier berechnet und als Mittelwert mit Standardabweichung pro Gruppe (n = 6 oder n = 12) angegeben. Sind die Substanzeffekte im Vergleich zur Lösungsmittel-behandelten Gruppe signifikant, wird der mittels t-Test ermittelte p-Wert hinzugefügt (* p ≤0.05; ** p ≤0.01; *** p ≤0.005).

| **Beispiel Nr.** | **% HDL-Anstieg nach 24 h (Dosis: 2 x 10 mg/kg)** |
|---|---|
| 1 | 20 |

### B-II.3. Messung der in vivo-Wirksamkeit an transgenen hCETP-Mäusen

Zur Bestimmung der oralen Wirkung auf Lipoproteine und Triglyceride wird transgenen Mäusen [Dinchuk et al., BBA, 1295-1301 (1995)] Testsubstanz mit der Schlundsonde verabreicht. Vor Versuchsbeginn wird den Mäusen retroorbital Blut entnommen, um Cholesterin und Triglyceride im Serum zu bestimmen. Das Serum wird, wie oben für Hamster beschrieben, durch Inkubation bei 4°C über Nacht und anschließende Zentrifugation bei 6.000 g gewonnen. Nach drei Tagen wird den Mäusen wieder Blut entnommen, um Lipoproteine und Triglyceride erneut zu bestimmen. Die Veränderungen der gemessenen Parameter werden als prozentuale Veränderung gegenüber dem Ausgangswert ausgedrückt.

| **Beispiel Nr.** | **% HDL-Anstieg nach 3 d (Dosis: 3 x 3 mg/kg)** |
|---|---|
| 1 | 85 |

### C. Ausführungsbeispiele für pharmazeutische Zusammensetzungen (b)

Die erfindungsgemäße Verbindung kann folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 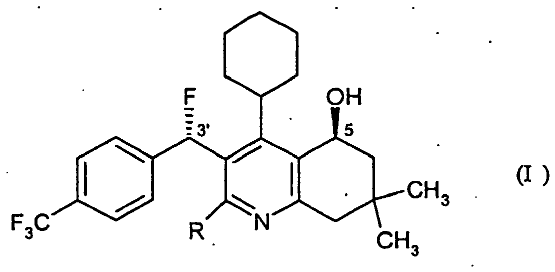 in denen R für Cyclopentyl oder iso-Propyl steht, sowie ihre Salze, Solvate und Solvate der Salze.

2. Verbindung der Formel (Ia) 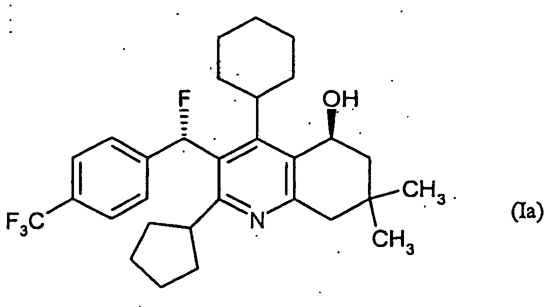 sowie ihre Salze, Solvate und Solvate der Salze.

3. Verbindung der Formel (Ib) 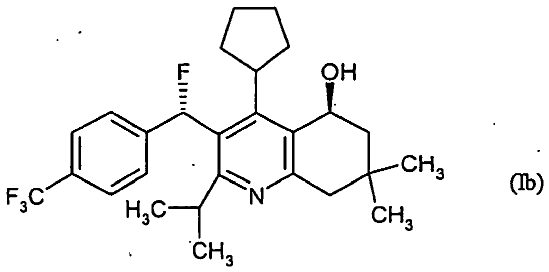 sowie ihre Salze, Solvate und Solvate der Salze.

4. Verbindungen wie in einem der Ansprüchen 1 bis 3 definiert, zur Behandlung und/oder Prävention von Krankheiten.

5. Verwendung der Verbindungen wie in einem der Ansprüchen 1 bis 3 definiert, zur Herstellung eines Arzneimittels zur primären und/oder sekundären Prävention von koronaren Herzerkrankungen.

6. Verwendung der Verbindungen wie in einem der Ansprüchen 1 bis 3 definiert, zur Herstellung eines Arzneimittels zur Behandlung und/oder Prävention von Hypolipoproteinämien, Dyslipidämien, Hypertriglyceridämien, Hyperlipidämien, Hypercholesterolämien, Arteriosklerose, Restenose, Fettsucht (Adipositas), Fettleibigkeit (Obesitas), Diabetes, Schlaganfall und der Alzheimer'schen Krankheit.

7. Arzneimittel enthaltend die Verbindungen, wie in einem der Ansprüchen 1 bis 3 definiert, in Kombination mit einem inerten, nicht-toxischen, pharmazeutisch geeigneten Hilfsstoff.

8. Arzneimittel enthaltend die Verbindungen, wie in einem der Ansprüchen 1 bis 3 definiert, in Kombination mit einem oder mehreren weiteren Wirkstoffen ausgewählt aus der Gruppe bestehend aus Antidiabetika, Thrombozytenaggregationshemmer, Antikoagulantien, Calcium-Antagonisten, Angiotensin AII-Antagonisten, ACE-Hemmer, beta-Blocker, Phosphodiesterase-Inhibitoren, Stimulatoren der löslichen Guanylatcyclase, cGMP-Enhancer, Diuretika, Thyroidrezeptor-Agonisten, HMG-CoA-Reduktase-Inhibitoren, Squalensynthase-Inhibitoren, Squalenepoxidase-Inhibitoren, Oxidosqüalencyclase-Inhibitoren, ACAT-Inhibitoren, MTP-Inhibitoren, PPAR-Agonisten, Fibrate, Lipase-Inhibitoren, Cholesterinabsorptionshemmer, Gallensäure-Reabsorptionshemmer, polymeren Gallensäureadsorber und Lipoprotein(a)-Antagonisten.

9. Arzneimittel nach Anspruch 7 oder 8 zur primären und/oder sekundären Prävention von koronaren Herzerkrankungen.

10. Arzneimittel nach Anspruch 7 oder 8 zur Behandlung und/oder Prävention von Hypolipoproteinämien, Dyslipidämien, Hypertriglyceridämien, Hyperlipidämien, Hypercholesterolämien, Arteriosklerose, Restenose, Fettsucht (Adipositas), Fettleibigkeit (Obesitas), Diabetes, Schlaganfall und der Alzheimer'schen Krankheit.

11. Verfahren zur Herstellung der Verbindung der Formel (Ia), wie in Anspruch 2 definiert, **dadurch gekennzeichnet, dass** man die Verbindung der Formel (IIa) 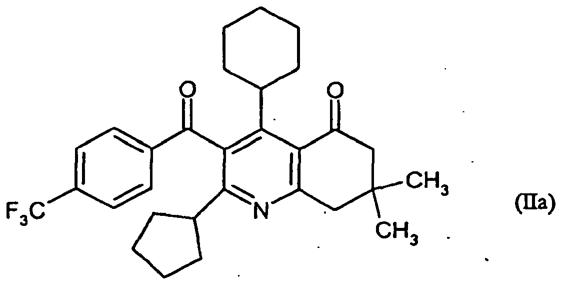 zunächst durch eine asymmetrische Reduktion in die Verbindung der Formel (IIIa) 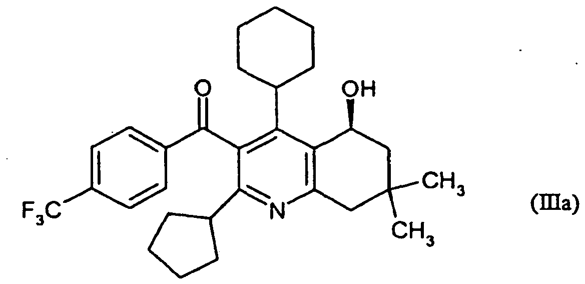 überführt, diese dann entweder
[A] durch Einführung einer Hydroxy-Schutzgruppe zu einer Verbindung der Formel (IVa) 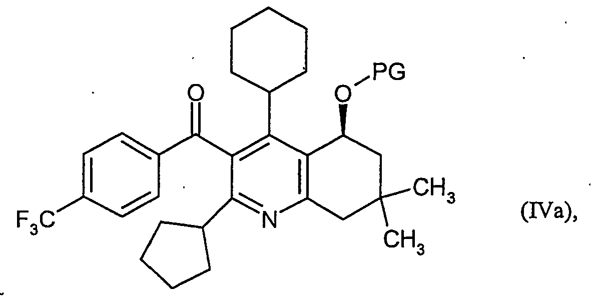 in welcher
PG für eine Hydroxy-Schutzgruppe, vorzugsweise für einen Rest der Formel -SiR¹R²R³ steht, worin
R¹, R² und R³ gleich oder verschieden sind und (C₁-C₄)-Alkyl bedeuten,
umsetzt und anschließend über eine diastereoselektive Reduktion in eine Verbindung der Formel (Va) 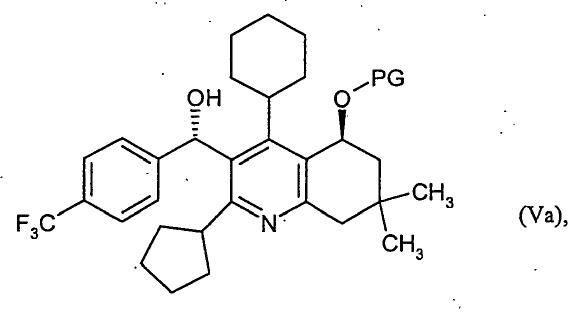 in welcher PG die oben angegebene Bedeutung hat,
überführt
oder
[B] zunächst diastereoselektiv zu der Verbindung der Formel (VIa) 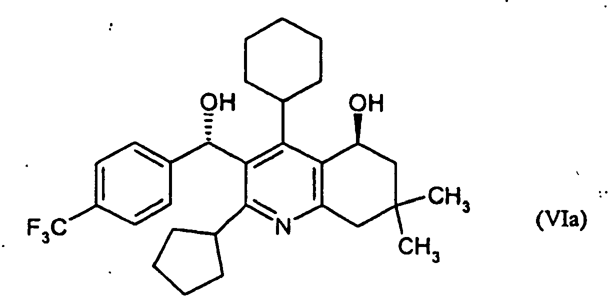 reduziert und diese dann durch regioselektive Einführung der Hydroxy-Schutzgruppe PG in eine Verbindung der Formel (V) überführt, anschließend die Verbindung der Formel (Va) mit Hilfe eines Fluorierungsmittels zu einer Verbindung der Formel (VIIa) 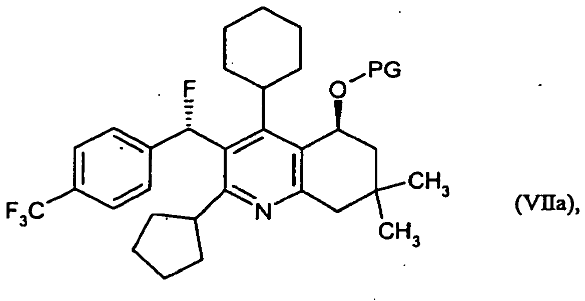 in welcher PG die oben angegebene Bedeutung hat,
umsetzt und dann die Hydroxy-Schutzgruppe PG nach üblichen Methoden unter Erhalt der Verbindung der Formel (Ia) wieder abspaltet
und die Verbindung der Formel (Ia) gegebenenfalls mit den entsprechenden (i) Lösungsmitteln und/oder (ii) Basen oder Säuren zu ihren Solvaten, Salzen und/oder Solvaten der Salze umsetzt.

12. Verfahren zur Herstellung der Verbindung der Formel (Ib), wie in Anspruch 3 definiert, **dadurch gekennzeichnet, dass** man die Verbindung der Formel (IIb) 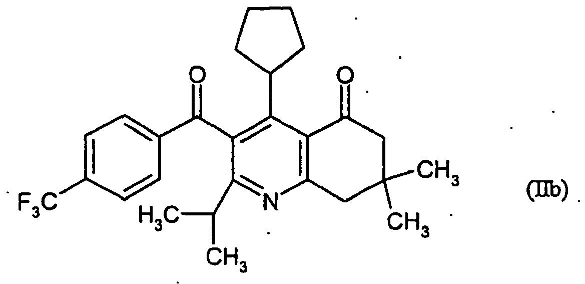 zunächst durch eine asymmetrische Reduktion in die Verbindung der Formel (IIIb) 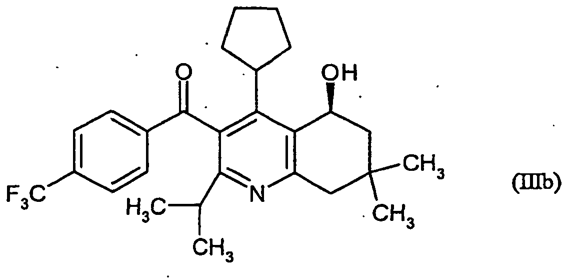 überführt, diese dann entweder
[A] durch Einführung einer Hydroxy-Schutzgruppe zu einer Verbindung der Formel (IVb) 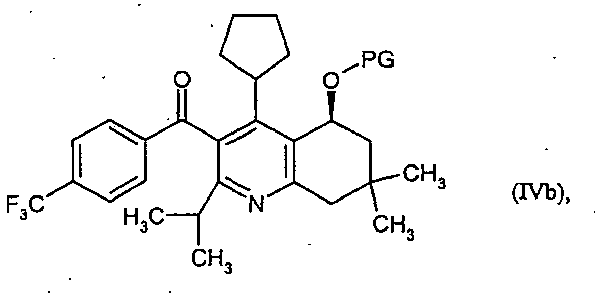 in welcher
PG für eine Hydroxy-Schutzgruppe, vorzugsweise für einen Rest der Formel -SiR¹R²R³ steht, worin
R¹, R² und R³ gleich oder verschieden sind und (C₁-C₄)-Alkyl bedeuten,
umsetzt und anschließend über eine diastereoselektive Reduktion in eine Verbindung der Formel (Vb) 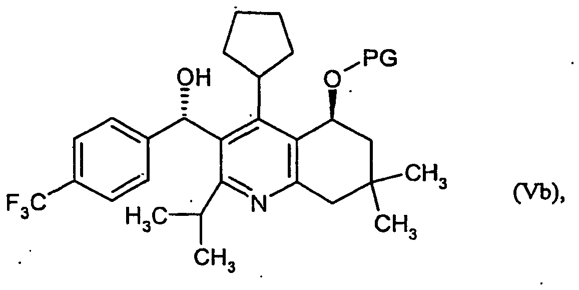 in welcher PG die oben angegebene Bedeutung hat,
überführt
oder
[B] zunächst diastereoselektiv zu der Verbindung der Formel (VIb) 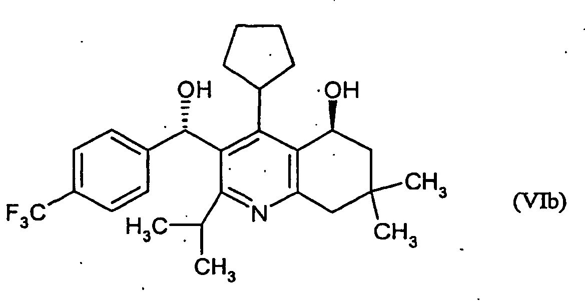 reduziert und diese dann durch regioselektive Einführung der Hydroxy-Schutzgruppe PG in eine Verbindung der Formel (Vb) überführt,
anschließend die Verbindung der Formel (Vb) mit Hilfe eines Fluorierungsmittels zu einer Verbindung der Formel (VIIb) 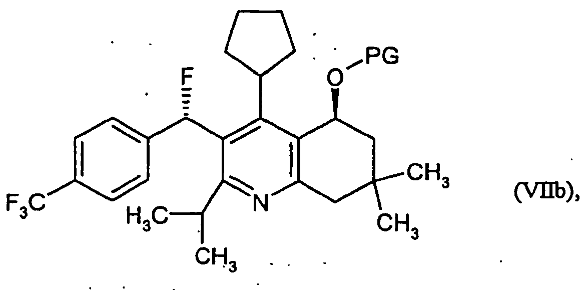 in welcher PG die oben angegebene Bedeutung hat,
umsetzt und dann die Hydroxy-Schutzgruppe PG nach üblichen Methoden unter Erhalt der Verbindung der Formel (Ib) wieder abspaltet
und die Verbindung der Formel (Ib) gegebenenfalls mit den entsprechenden (i) Lösungsmitteln und/oder (ii) Basen oder Säuren zu ihren Solvaten, Salzen und/oder Solvaten der Salze umsetzt.

## Claims

1. Compound of the formula (I) 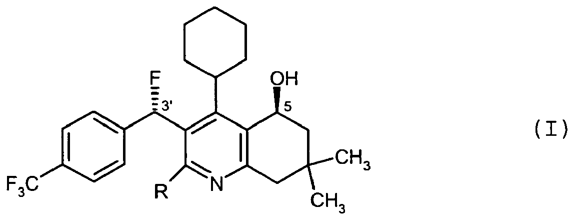 in which R represents cyclopentyl or isopropyl, and its salts, solvates and solvates of the salts.

2. Compound of the formula (Ia) 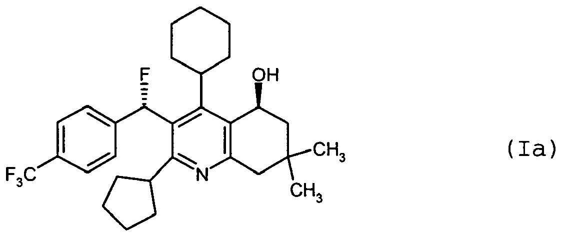 and its salts, solvates and solvates of the salts.

3. Compound of the formula (Ib) 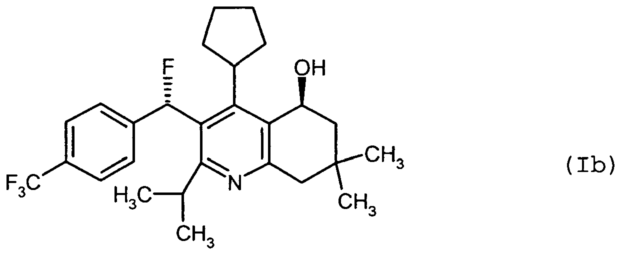 and its salts, solvates and solvates of the salts.

4. Compounds as defined in any of Claims 1 to 3 for the treatment and/or prevention of diseases.

5. Use of the compounds as defined in any of Claims 1 to 3 for preparing a medicament for the primary and/or secondary prevention of coronary heart disease.

6. Use of the compounds as defined in any of Claims 1 to 3 for preparing a medicament for the treatment and/or prevention of hypolipoproteinaemias, dyslipidaemias, hypertriglyceridaemias, hyperlipidaemias, hypercholesterolaemias, arteriosclerosis, restenosis, adiposity, obesity, diabetes, stroke and Alzheimer's disease.

7. Medicament, comprising the compounds as defined in any of Claims 1 to 3 in combination with an inert nontoxic pharmaceutically suitable auxiliary.

8. Medicament, comprising the compounds as defined in any of Claims 1 to 3 in combination with one or more further active compounds selected from the group consisting of antidiabetics, platelet aggregation inhibitors, anticoagulants, calcium antagonists, angiotensin AII antagonists, ACE inhibitors, beta blockers, phosphodiesterase inhibitors, stimulators of soluble guanylate cyclase, cGMP enhancers, diuretics, thyroid receptor agonists, HMG-CoA reductase inhibitors, squalene synthase inhibitors, squalene epoxidase inhibitors, oxidosqualene cyclase inhibitors, ACAT inhibitors, MTP inhibitors, PPAR agonists, fibrates, lipase inhibitors, cholesterol absorption inhibitors, bile acid reabsorption inhibitors, polymeric bile acid adsorbers and lipoprotein(a) antagonists.

9. Medicaments according to Claim 7 or 8 for the primary and/or secondary prevention of coronary heart disease.

10. Medicament according to Claim 7 or 8 for the treatment and/or prevention of hypolipoproteinaemias, dyslipidaemias, hypertriglyceridaemias, hyperlipidaemias, hypercholesterolaemias, arteriosclerosis, restenosis, adiposity, obesity, diabetes, stroke and Alzheimer's disease.

11. Process for preparing the compound of the formula (Ia) as defined in Claim 2, **characterized in that** the compound of the formula (IIa) 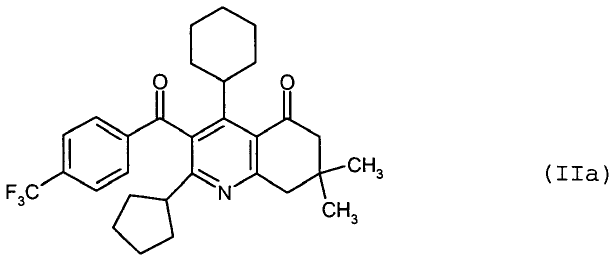 is initially, by asymmetric reduction, converted into the compound of the formula (IIIa) 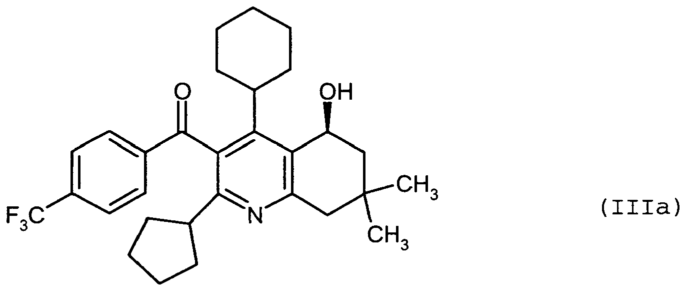 which is then either
[A] by introduction of a hydroxyl protective group reacted to give a compound of the formula (IVa) 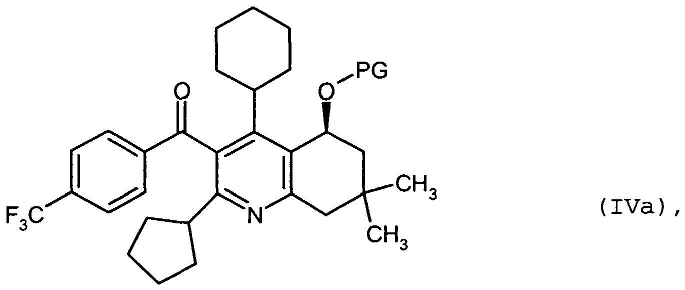 in which
PG represents a hydroxyl protective group, preferably a radical of the formula - SiR¹R²R³, in which R¹, R² and R³ are identical or different and represent (C₁-C₄) alkyl,
and then, by diastereoselective reduction, converted into a compound of the formula (Va) 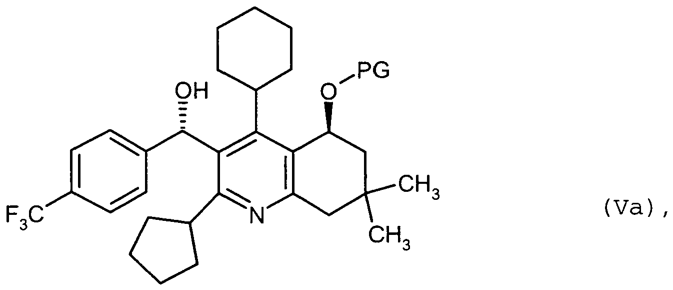 in which PG is as defined above,
or
[B] initially reduced diastereoselectively to give the compound of the formula (VIa) 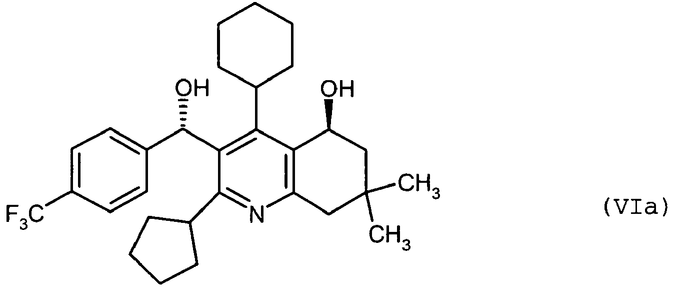 which is then, by regioselective introduction of the hydroxyl protective group PG, converted into a compound of the formula (Va),
the compound of the formula (Va) is then, using a fluorinating agent, reacted to give a compound of the formula (VIIa) 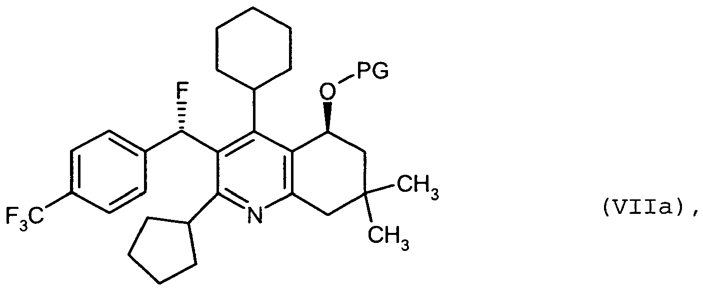 in which PG is as defined above,
and the hydroxyl protective group PG is then cleaved off by customary methods giving the compound of the formula (Ia)
and the compound of the formula (Ia) is, if appropriate, converted with the appropriate (i) solvents and/or (ii) bases and/or acids into its solvates, salts and/or solvates of the salts.

12. Process for preparing the compound of the formula (Ib) as defined in Claim 3, **characterized in that** the compound of the formula (IIb) 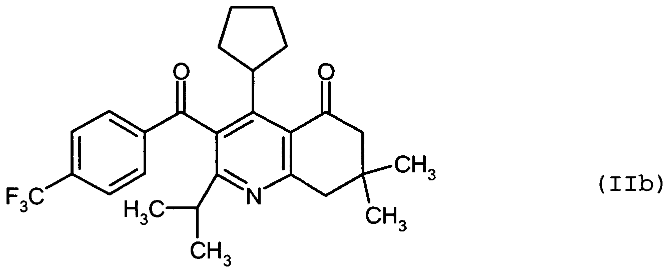 is initially, by asymmetric reduction, converted into the compound of the formula (IIIb) 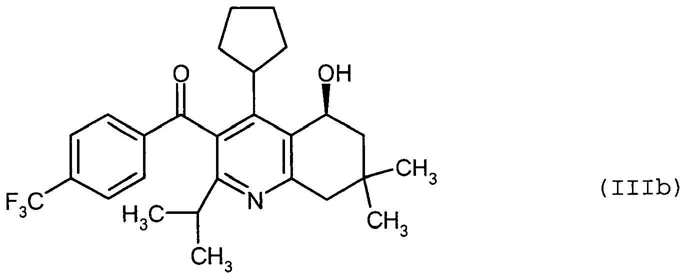 which is then either
[A] by introduction of a hydroxyl protective group reacted to give a compound of the formula (IVb) 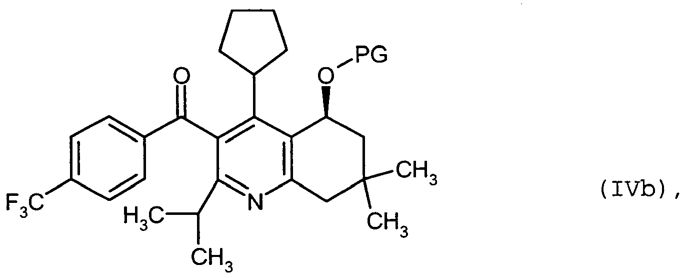 in which
PG represents a hydroxyl protective group, preferably a radical of the formula - SiR¹R²R³, in which
R¹, R² and R³ are identical or different and represent (C₁-C₄) alkyl,
and then, by diastereoselective reduction, converted into a compound of the formula (Vb) 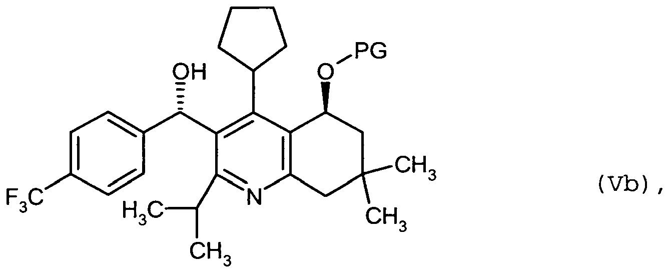 in which PG is as defined above,
or
[B] initially reduced diastereoselectively to give the compound of the formula (VIb) 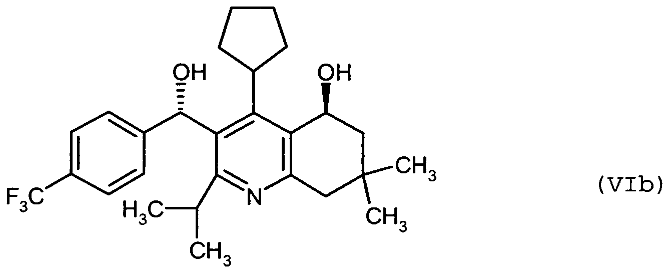 which is then, by regioselective introduction of the hydroxyl protective group PG, converted into a compound of the formula (Vb),
the compound of the formula (Vb) is then, using a fluorinating agent, reacted to give a compound of the formula (VIIb) 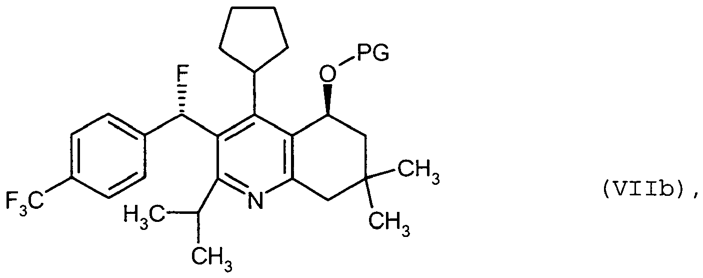 in which PG is as defined above,
and the hydroxyl protective group PG is then cleaved off by customary methods giving the compound of the formula (Ib)
and the compound of the formula (Ib) is, if appropriate, converted with the appropriate (i) solvents and/or (ii) bases and/or acids into its solvates, salts and/or solvates of the salts.

## Revendications

1. Composé de formule (I) 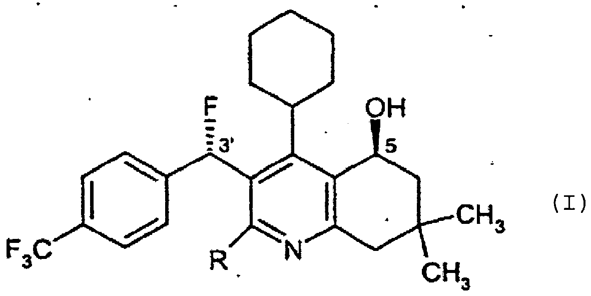 dans laquelle R représente le groupe cyclopentyle ou isopropyle, ainsi que ses sels, produits de solvatation et produits de solvatation des sels.

2. Composé de formule (Ia) 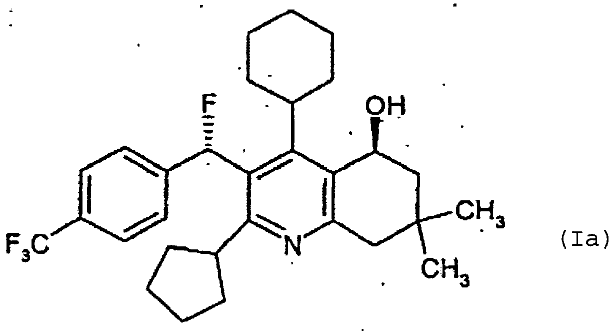 ainsi que ses sels, produits de solvatation et produits de solvatation des sels.

3. Composé de formule (Ib) 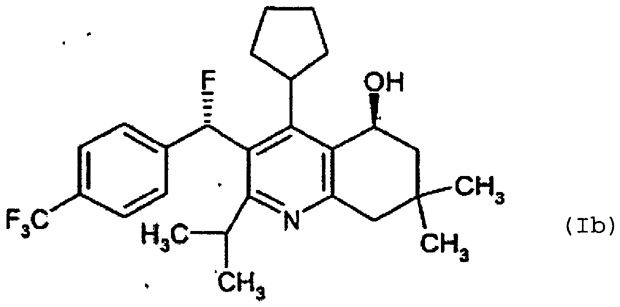 ainsi que ses sels, produits de solvatation et produits de solvatation des sels.

4. Composés tels que définis dans l'une quelconque des revendications 1 à 3, pour le traitement et/ou la prévention de maladies.

5. Utilisation des composés tels que définis dans l'une quelconque des revendications 1 à 3, pour la fabrication d'un médicament destiné à la prévention primaire et/ou secondaire de cardiopathies coronariennes.

6. Utilisation des composés tels que définis dans l'une quelconque des revendications 1 à 3, pour la fabrication d'un médicament destiné au traitement et/ou à la prévention d'hypolipoprotéinémies, de dyslipidémies, d'hypertriglycéridémies, d'hyperlipidémies, d'hypercholestérolémies, de l'artériosclérose, de la resténose, de l'adiposité (adipositas), de l'obésité (obesitas), du diabète, de l'accident vasculaire cérébral et de la maladie d'Alzheimer.

7. Médicament contenant les composés tels que définis dans l'une quelconque des revendications 1 à 3, en association avec un adjuvant inerte, non toxique, pharmaceutiquement approprié.

8. Médicament contenant les composés tels que définis dans l'une quelconque des revendications 1 à 3, en association avec une ou plusieurs substances actives choisies dans le groupe constitué par les antidiabétiques, inhibiteurs de l'agrégation thrombocytaire, anticoagulants, antagonistes du calcium, antagonistes de l'angiotensine AII, inhibiteurs d'ACE, bêta-bloquants, inhibiteurs de phosphodiestérase, stimulants de la guanylate cyclase soluble, activateurs de cGMP, diurétiques, agonistes des récepteurs thyroïdiens, inhibiteurs de HMG-CoA-réductase, inhibiteurs de squalène synthase, inhibiteurs de squalène époxydase, inhibiteurs d'oxydosqualène cyclase, inhibiteurs d'ACAT, inhibiteurs de MTP, agonistes de PPAR, fibrates, inhibiteurs de lipases, inhibiteurs d'absorption du cholestérol, inhibiteurs de réabsorption des acides biliaires, adsorbeurs polymères d'acides biliaires et antagonistes de la lipoprotéine (a).

9. Médicament selon la revendication 7 ou 8, destiné à la prévention primaire et/ou secondaire de cardiopathies coronariennes.

10. Médicament selon la revendication 7 ou 8, destiné au traitement et/ou à la prévention d'hypolipoprotéinémies, de dyslipidémies, d'hypertriglycéridémies, d'hyperlipidémies, d'hypercholestérolémies, de l'artériosclérose, de la resténose, de l'adiposité (adipositas), de l'obésité (obesitas), du diabète, de l'accident vasculaire cérébral et de la maladie d'Alzheimer.

11. Procédé pour la préparation du composé de formule (Ia), tel que défini dans la revendication 2, **caractérisé en ce que** d'abord on convertit le composé de formule (IIa) 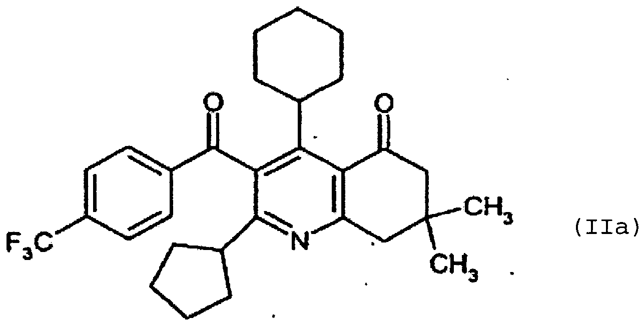 par une réduction asymétrique, en le composé de formule (IIIa) 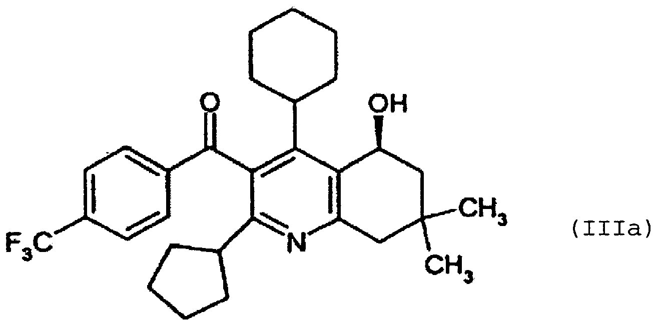 puis soit
[A] on convertit ce dernier, par introduction d'un groupe protecteur de fonction hydroxy, en un composé de formule (IVa) 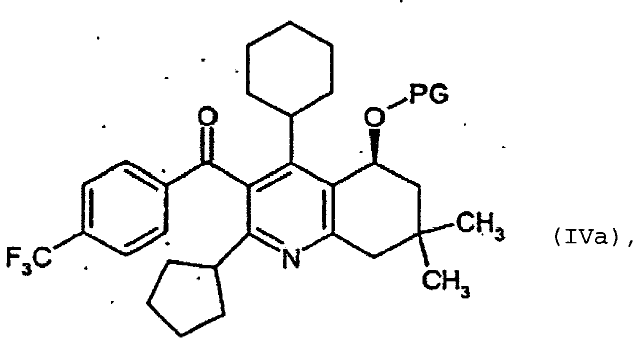 dans laquelle
PG représente un groupe protecteur de fonction hydroxy, de préférence un radical de formule -SiR¹R²R³, dans lequel R¹, R² et R³ sont identiques ou différents et représentent un groupe alkyle en C₁-C_{4,}
et ensuite, par réduction diastéréosélective, en un composé de formule (Va) 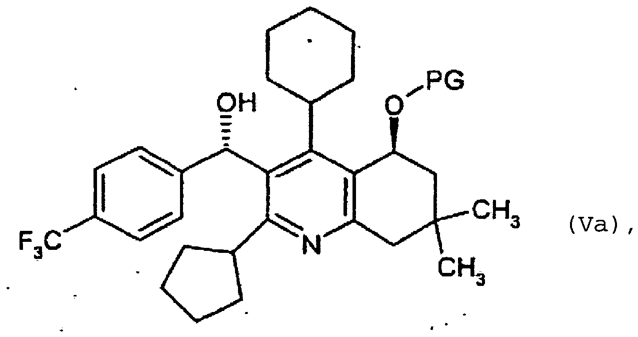 dans laquelle PG est tel que défini ci-dessus, soit
[B] d'abord on le réduit, par réduction diastéréosélective, en un composé de formule (VIa) 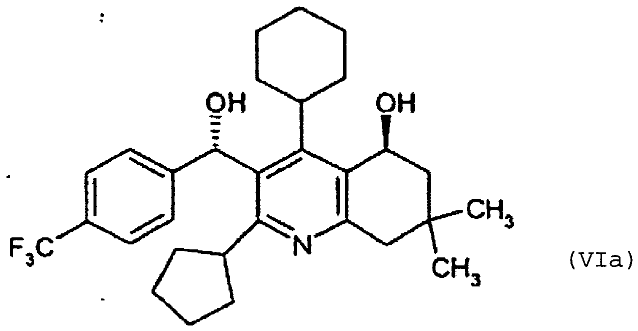 et ensuite on convertit ce dernier, par introduction régiosélective du groupe protecteur de fonction hydroxy PG, en un composé de formule (V),
puis on convertit le composé de formule (Va), à l'aide d'un agent de fluoration, en un composé de formule (VIIa) 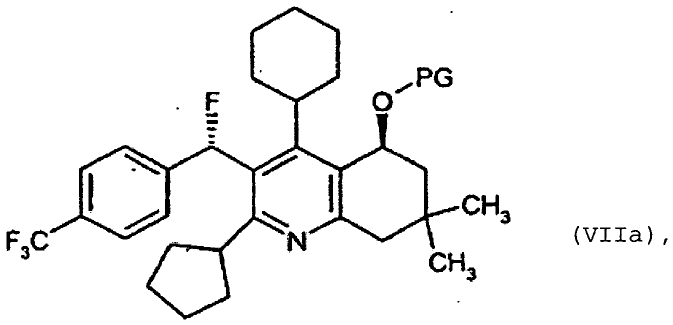 dans laquelle PG a la signification indiquée plus haut,
et ensuite on élimine à nouveau le groupe protecteur de fonction hydroxy PG, selon des méthodes usuelles, avec obtention du composé de formule (Ia)
et éventuellement on convertit le composé de formule (Ia), à l'aide (i) des solvants correspondants et/ou (ii) des bases correspondantes ou des acides correspondants, en ses produits de solvatation, sels et/ou produits de solvatation des sels.

12. Procédé pour la préparation du composé de formule (Ib), tel que défini dans la revendication 3, **caractérisé en ce que** d'abord on convertit le composé de formule (IIb) 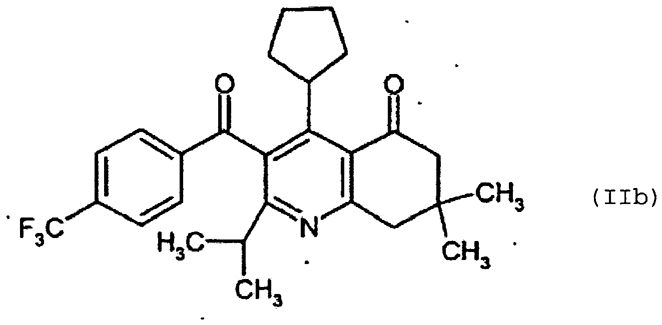 par une réduction asymétrique, en le composé de formule (IIIb) 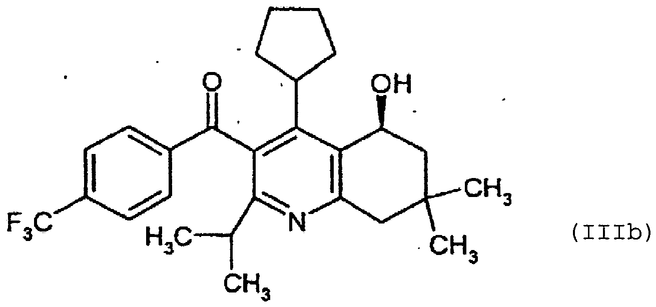 puis soit
[A] on convertit ce dernier, par introduction d'un groupe protecteur de fonction hydroxy, en un composé de formule (IVb) 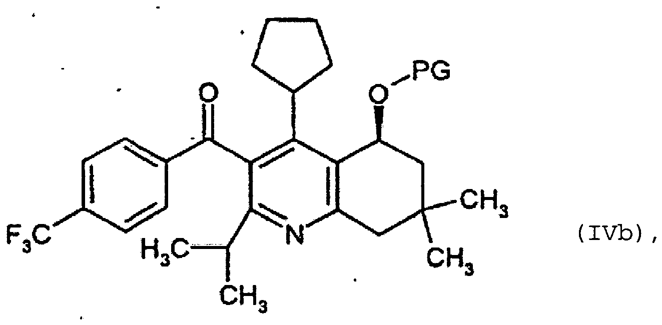 dans laquelle
PG représente un groupe protecteur de fonction hydroxy, de préférence un radical de formule -SiR¹R²R³, dans lequel R¹, R² et R³ sont identiques ou différents et représentent un groupe alkyle en C₁-C_{4,}
et ensuite, par une réduction diastéréosélective, en un composé de formule (Vb) 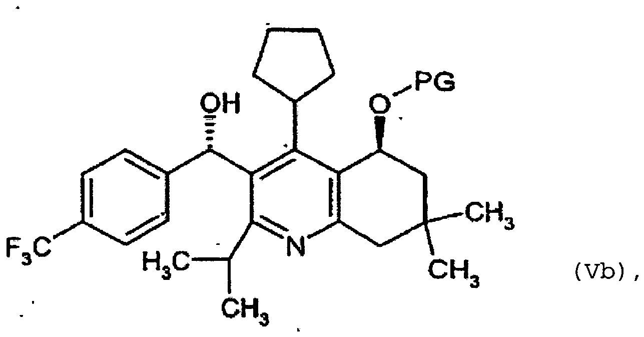 dans laquelle PG est tel que défini ci-dessus, soit
[B] d'abord on le réduit par réduction diastéréosélective, en le composé de formule (VIb) 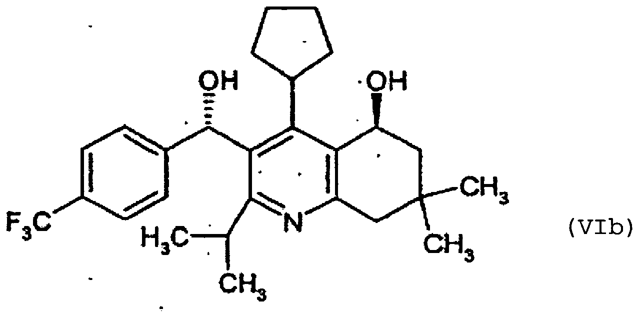 et ensuite on convertit ce dernier, par introduction régiosélective du groupe protecteur de fonction hydroxy PG, en un composé de formule (Vb),
puis on convertit le composé de formule (Vb), à l'aide d'un agent de fluoration, en un composé de formule (VIIb) 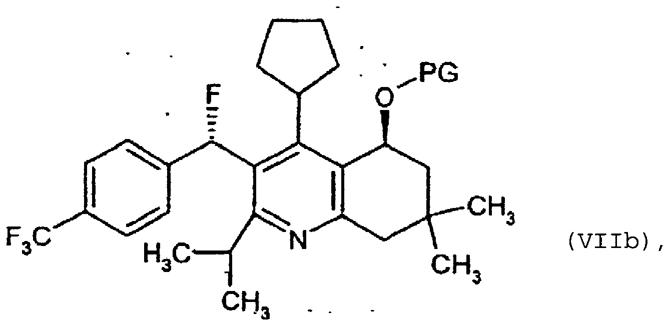 dans laquelle PG a la signification indiquée plus haut,
et ensuite on élimine à nouveau le groupe protecteur de fonction hydroxy PG, selon des méthodes usuelles, avec obtention du composé de formule (Ib)
et éventuellement on convertit le composé de formule (Ib), à l'aide (i) des solvants correspondants et/ou (ii) des bases correspondantes ou des acides correspondants, en ses produits de solvatation, sels et/ou produits de solvatation des sels.
